(19) Europäisches Patentamt
European Patent Office
Office européen des brevets

(11) EP 0 764 647 A1

(12) **EUROPÄISCHE PATENTANMELDUNG**

(43) Veröffentlichungstag:
**26.03.1997 Patentblatt 1997/13**

(51) Int. Cl.[6]: **C07D 473/00**, C07D 473/06, C07D 473/08, A61K 31/52

(21) Anmeldenummer: **96114577.8**

(22) Anmeldetag: **12.09.1996**

(84) Benannte Vertragsstaaten:
**AT BE CH DE DK ES FI FR GB GR IE IT LI LU MC NL PT SE**

(30) Priorität: **25.09.1995 DE 19535504**

(71) Anmelder: **BAYER AG**
**51368 Leverkusen (DE)**

(72) Erfinder:
- **Connell, Richard, Dr.**
  **Trumbull, CT 06611 (US)**
- **Goldmann, Siegfried, Dr.**
  **42327 Wuppertal (DE)**
- **Müller, Ulrich, Dr.**
  **42111 Wuppertal (DE)**
- **Lohmer, Stefan, Dr.**
  **20132 Milano (IT)**
- **Bischoff, Hilmar, Dr.**
  **42113 Wuppertal (DE)**
- **Denzer, Dirk, Dr.**
  **42115 Wuppertal (DE)**
- **Grützmann, Rudi, Dr.**
  **42657 Solingen (DE)**
- **Wohlfeil, Stefan, Dr.**
  **40721 Hilden (DE)**

(54) **Substituierte Xanthine**

(57) Substituierte Xanthine werden hergestellt durch Umsetzung der geeigneten unsubstituierten Xanthine mit Halogenmethylphenylessigsäuren und anschließende Reaktion der Carbonester bzw. -säuren mit Phenylglycinolamin. Die substituierten Xanthine eignen sich als Wirkstoffe in Arzneimitteln, insbesondere in antiatherosklerotischen Arzneimitteln.

EP 0 764 647 A1

## Beschreibung

Die vorliegende Erfindung betrifft substituierte Xanthine, Verfahren zu ihrer Herstellung und ihre Verwendung als Arzneimittel, insbesondere als antiatherosklerotische Arzneimittel.

Es ist bekannt, daß erhöhte Blutspiegel von Triglyceriden (Hypertriglyceridämie) und Cholesterin (Hypercholesterinämie) mit der Genese von atherosklerotischen Gefäßwand-Veränderungen und koronaren Herzkrankheiten assoziiert sind.

Ein deutlich erhöhtes Risiko für die Entwicklung koronarer Herzerkrankungen liegt darüberhinaus vor, wenn diese beiden Risikofaktoren kombiniert auftreten, was wiederum mit einer Überproduktion an Apoliprotein B-100 einhergeht. Es ist daher nach wie vor ein starkes Bedürfnis, wirksame Arzneimittel zur Bekämpfung der Atherosklerose sowie koronarer Herzkrankheiten zur Verfügung zu stellen.

Die vorliegende Erfindung betrifft substituierte Xanthine der allgemeinen Formel (I)

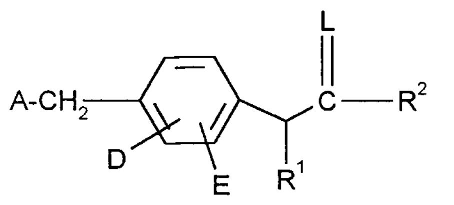
(I)

in welcher

A für einen Rest der Formel

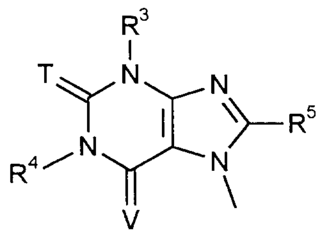
oder
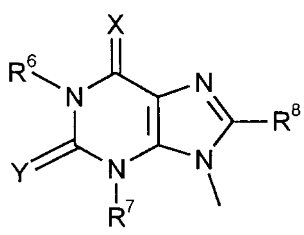
steht,

worin

$R^3$, $R^4$, $R^6$ und $R^7$ gleich oder verschieden sind und Wasserstoff, Cycloalkyl mit 3 bis 7 Kohlenstoffatomen oder Aryl mit 6 bis 10 Kohlenstoffatomen bedeuten,
oder geradkettiges oder verzweigtes Alkyl oder Alkenyl mit jeweils bis zu 8 Kohlenstoffatomen bedeuten, die gegebenenfalls durch Halogen, Hydroxy oder Aryl mit 6 bis 10 Kohlenstoffatomen substituiert sind,

T, V, X und Y gleich oder verschieden sind und
ein Sauerstoff- oder Schwefelatom bedeuten,

$R^5$ und $R^8$ gleich oder verschieden sind und
Wasserstoff, Halogen, Cycloalkyl mit 3 bis 8 Kohlenstoffatomen oder geradkettiges oder verzweigtes Alkyl oder Alkenyl mit jeweils bis zu 8 Kohlenstoffatomen bedeuten, die gegebenenfalls durch Cycloalkyl mit 3 bis 8 Kohlenstoffatomen, oder durch einen 5- bis 6-gliedrigen, aromatischen, gegebenenfalls benzokondensierten Heterocyclus mit bis zu 3 Heteroatomen aus der Reihe S, N und/oder O, oder durch Aryl mit 6 bis 10 Kohlenstoffatomen substituiert sind, wobei die Cyclen ihrerseits bis zu 3-fach gleich oder verschieden durch einen 5- bis 6-gliedrigen aromatischen Heterocyclus mit bis zu 3 Heteroatomen aus der Reihe S, H und/oder O, oder durch Phenyl, Benzyl, Halogen, Hydroxy, Carboxyl oder durch geradkettiges oder verzweigtes Alkyl, Alkoxy oder Alkoxycarbonyl mit jeweils bis zu 6 Kohlenstoffatomen substituiert sein können, oder
Aryl mit 6 bis 10 Kohlenstoffatomen oder einen 5- bis 7-gliedrigen aromatischen, gegebenenfalls benzokondensierten Heterocyclus mit bis zu 3 Heteroatomen aus der Reihe S, N und/oder O bedeuten, die gegebenenfalls bis zu 3-fach gleich oder verschieden

durch Halogen, Phenyl, Trifluormethyl, Hydroxy, Carboxyl oder durch geradkettiges oder verzweigtes Alkyl, Alkoxy oder Alkoxycarbonyl mit jeweils bis zu 6 Kohlenstoffatomen oder durch eine Gruppe der Formel $-(CO)_a-NR^9R^{10}$substituiert sind, worin

a — eine Zahl 0 oder 1 bedeutet,

$R^9$ und $R^{10}$ — gleich oder verschieden sind und Wasserstoff, Phenyl oder geradkettiges oder verzweigtes Alkyl oder Acyl mit jeweils bis zu 5 Kohlenstoffatomen bedeuten,

D und E — gleich oder verschieden sind und für Wasserstoff, Halogen, Trifluormethyl, Hydroxy, Carboxyl oder für geradkettiges oder verzweigtes Alkyl, Alkoxy oder Alkoxycarbonyl mit jeweils bis zu 6 Kohlenstoffatomen stehen,

$R^1$ — für Wasserstoff oder Cycloalkyl mit 3 bis 8 Kohlenstoffatomen steht, oder für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen, Phenyl oder durch einen 5- bis 6-gliedrigen aromatischen Heterocyclus mit bis zu 3 Heteroatomen aus der Reihe S, N und/oder O substituiert sind, oder für Phenyl oder einen 5- bis 6-gliedrigen aromatischen Heterocylcus mit bis zu 3 Heteroatomen aus der Reihe S, N und/oder O steht, wobei die Ringsysteme gegebenenfalls bis zu 3-fach gleich oder verschieden durch Halogen, Phenyl, Trifluormethyl oder geradkettiges oder verzweigtes Alkyl oder Alkoxy mit jeweils bis zu 5 Kohlenstoffatomen, Hydroxy oder durch eine Gruppe der Formel $-NR^{11}R^{12}$ substituiert sind, worin

$R^{11}$ und $R^{12}$ — die oben angegebene Bedeutung von $R^9$ und $R^{10}$ haben und mit dieser gleich oder verschieden sind,

L — für ein Sauerstoff- oder Schwefelatom steht,

$R^2$ — für Mercapto, Hydroxy, geradkettiges oder verzweigtes Alkoxy mit bis zu 8 Kohlenstoffatomen oder für die Gruppe der Formel

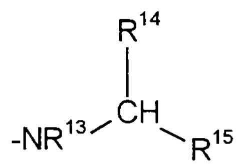

steht, worin

$R^{13}$ — Wasserstoff oder geradkettiges oder verzweigtes Alkyl mit bis zu 4 Kohlenstoffatomen bedeutet,

$R^{14}$ — Wasserstoff, Phenyl oder einen 5- bis 6-gliedrigen aromatischen Heterocyclus mit bis zu 3 Heteroatomen aus der Reihe S, N und/oder O bedeutet,

$R^{15}$ — Wasserstoff oder geradkettiges oder verzweigtes Alkyl mit bis zu 8 Kohlenstoffatomen bedeutet, das gegebenenfalls durch Hydroxy substituiert ist,

und deren Salze.

Die erfindungsgemäßen substituierten Xanthine können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch

unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten oder ungesättigten 5- bis 7-gliedrigen, vorzugsweise 5- bis 6-gliedrigen Heterocyclus, der bis zu 3 Heteroatome aus der Reihe S, N und/oder O enthalten kann. Beispielsweise seien genannt: Indolyl, Chinolyl, Benzo[b]thiophen, Benzo[b]furanyl, Pyridyl, Thienyl, Furyl, Pyrrolyl, Thiazolyl, Oxazolyl, Imidazolyl, Morpholinyl oder Piperidyl. Bevorzugt sind Chinolyl, Furyl, Pyridyl und Thienyl.

Bevorzugt sind Verbindungen der allgemeinen Formel (I),
in welcher

A für einen Rest der Formel

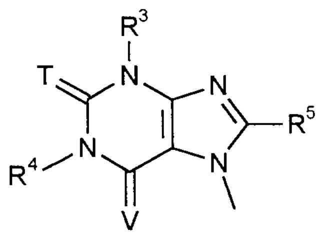 oder 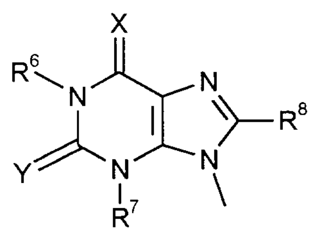 steht,

worin

$R^3$, $R^4$, $R^6$ und $R^7$ gleich oder verschieden sind und
Wasserstoff, Phenyl, Cyclopropyl, Cyclopentyl oder Cyclohexyl bedeuten, oder geradkettiges oder verzweigtes Alkyl oder Alkenyl mit jeweils bis zu 6 Kohlenstoffatomen bedeuten, die gegebenenfalls durch Fluor, Chlor, Brom, Hydroxy oder Phenyl substituiert sind,

T, V, X und Y gleich oder verschieden sind und
ein Sauerstoff- oder Schwefelatom bedeuten,

$R^5$ und $R^8$ gleich oder verschieden sind und
Wasserstoff, Fluor, Chlor, Brom, Cyclopropyl, Cyclobutyl, Cyclopentyl, Cyclohexyl, Cycloheptyl oder für geradkettiges oder verzweigtes Alkyl oder Alkenyl mit jeweils bis zu 6 Kohlenstoffatomen bedeuten, die gegebenenfalls durch Cyclopropyl, Cyclopentyl, Cyclohexyl, Cycloheptyl, Naphthyl, Phenyl, Pyridyl, Thienyl oder Furyl substituiert sind, die ihrerseits bis zu 2-fach gleich oder verschieden durch Phenyl, Benzyl, Fluor, Chlor, Brom, Hydroxy oder durch geradkettiges oder verzweigtes Alkyl oder Alkoxy mit jeweils bis zu 4 Kohlenstoffatomen substituiert sein können, oder
Phenyl, Pyridyl, Thienyl oder Furyl bedeuten, die gegebenenfalls bis zu 2-fach gleich oder verschieden durch Fluor, Chlor, Brom, Phenyl, Trifluormethyl, Hydroxy oder durch geradkettiges oder verzweigtes Alkyl oder Alkoxy mit jeweils bis zu 4 Kohlenstoffatomen oder durch eine Gruppe der Formel $-(CO)_a-NR^9R^{10}$ substituiert sind,
worin

| | |
|---|---|
| a | eine Zahl 0 oder 1 bedeutet, |
| $R^9$ und $R^{10}$ | gleich oder verschieden sind und Wasserstoff, Phenyl oder geradkettiges oder verzweigtes Alkyl oder Acyl mit jeweils bis zu 4 Kohlenstoffatomen bedeuten, |
| D und E | gleich oder verschieden sind und für Wasserstoff, Fluor, Chlor, Brom, Trifluormethyl, Hydroxy oder geradkettiges oder verzweigtes Alkyl oder Alkoxy mit jeweils bis zu 4 Kohlenstofatomen stehen, |
| $R^1$ | für Wasserstoff, Cyclopropyl, Cyclobutyl, Cyclopentyl, Cyclohexyl, Cycloheptyl oder für geradkettiges oder verzweigtes Alkyl oder Alkenyl mit jeweils bis zu 6 Kohlenstoffatomen steht, die gegebenenfalls durch Cylcopropyl, Cyclopentyl, Cyclohexyl, Phenyl, Pyridyl oder Thienyl substituiert sind, oder für Phenyl, Pyridyl, Furyl oder Thienyl steht, die gegebenenfalls bis zu 2-fach gleich oder verschieden durch Fluor, Chlor, Brom, Phenyl, Trifluormethyl oder geradkettiges oder verzweigtes Alkyl oder Alkoxy mit jeweils bis zu 4 Kohlenstoffatomen oder durch eine Gruppe der Formel $-NR^{11}R^{12}$ substituiert sind, worin |
| $R^{11}$ und $R^{12}$ | die oben angegebene Bedeutung von $R^9$ und $R^{10}$ haben und mit dieser gleich oder verschieden sind, |
| L | für ein Sauerstoff- oder Schwefelatom steht, |
| $R^2$ | für Mercapto, Hydroxy, geradkettiges oder verzweigtes Alkoxy mit bis zu 6 Kohlenstoffatomen oder für die Gruppe der Formel 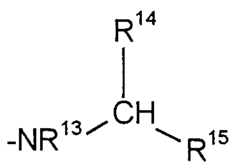 steht, worin |
| $R^{13}$ | Wasserstoff oder geradkettiges oder verzweigtes Alkyl mit bis zu 4 Kohlenstoffatomen bedeutet, |
| $R^{14}$ | Wasserstoff, Phenyl, Pyridyl, Furyl oder Thienyl bedeutet, |
| $R^{15}$ | Wasserstoff oder geradkettiges oder verzweigtes Alkyl mit bis zu 6 Kohlenstoffatomen bedeutet, das gegebenenfalls durch Hydroxy substituiert ist, |

und deren Salze.

Besonders bevorzugt sind Verbindungen der allgemeinen Formel (I), in welcher

| | |
|---|---|
| A | für einen Rest der Formel |

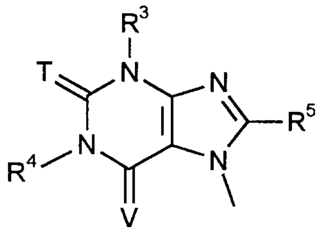

oder

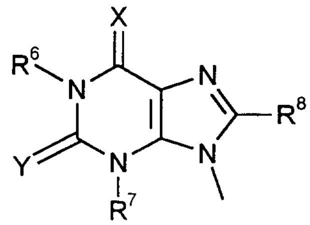

steht,
worin

| | |
|---|---|
| $R^3$, $R^4$, $R^6$ und $R^7$ | gleich oder verschieden sind und Wasserstoff, Phenyl, Cyclopropyl, Cyclopentyl oder Cyclohexyl bedeuten, oder geradkettiges oder verzweigtes Alkyl oder Alkenyl mit jeweils bis zu 5 Kohlenstoffatomen bedeuten, die gegebenenfalls durch Fluor, Chlor, Brom, Hydroxy oder Phenyl substituiert sind, |
| T, V, X und Y | gleich oder verschieden sind und ein Sauerstoff- oder Schwefelatom bedeuten, |
| $R^5$ und $R^8$ | gleich oder verschieden sind und Wasserstoff, Fluor, Chlor, Brom, Cyclopropyl, Cyclobutyl, Cyclopentyl, Cyclohexyl, Cycloheptyl oder für geradkettiges oder verzweigtes Alkyl oder Alkenyl mit jeweils bis zu 5 Kohlenstoffatomen bedeuten, die gegebenenfalls durch Cyclopropyl, Cyclopentyl, Cyclohexyl, Cycloheptyl, Phenyl, Pyridyl, Thienyl oder Furyl substituiert sind, die ihrerseits bis zu 2-fach gleich oder verschieden durch Phenyl, Benzyl, Fluor, Chlor, Brom, Hydroxy oder durch geradkettiges oder verzweigtes Alkyl oder Alkoxy mit jeweils bis zu 3 Kohlenstoffatomen substituiert sein können, oder Phenyl, Pyridyl, Thienyl oder Furyl bedeuten, die gegebenenfalls bis zu 2-fach gleich oder verschieden durch Fluor, Chlor, Brom, Phenyl, Trifluormethyl, Hydroxy oder durch geradkettiges oder verzweigtes Alkyl oder Alkoxy mit jeweils bis zu 3 Kohlenstoffatomen oder durch eine Gruppe der Formel $-(CO)_a-NR^9R^{10}$ substituiert sind, worin |
| a | eine Zahl 0 oder 1 bedeutet, |
| $R^9$ und $R^{10}$ | gleich oder verschieden sind und Wasserstoff Phenyl oder geradkettiges oder verzweigtes Alkyl oder Acyl mit jeweils bis zu 3 Kohlenstoffatomen bedeuten, |
| D und E | gleich oder verschieden sind und für Wasserstoff, Fluor, Chlor, Brom oder Trifluormethyl stehen, |
| $R^1$ | für Wasserstoff, Cyclopropyl, Cyclobutyl, Cyclopentyl, Cyclohexyl, Cycloheptyl oder für geradkettiges oder verzweigtes Alkyl oder Alkenyl mit jeweils bis zu 5 Kohlenstoffatomen steht, die gegebenenfalls durch Cyclopropyl, Cyclopentyl, Cyclohexyl, Phenyl, Pyridyl oder Thienyl substituiert sind, oder für Phenyl, Pyridyl, Furyl oder Thienyl steht, die gegebenenfalls bis zu 2-fach gleich oder verschieden durch Fluor, Chlor, Brom, Phenyl, Trifluormethyl oder geradkettiges oder verzweigtes Alkyl oder Alkoxy mit jeweils bis zu 3 Kohlenstoffatomen oder durch eine Gruppe der Formel $-NR^{11}R^{12}$ substituiert sind, worin |
| $R^{11}$ und $R^{12}$ | die oben angegebene Bedeutung von $R^9$ und $R^{10}$ haben und mit dieser gleich oder verschieden sind, |

L — für ein Sauerstoff- oder Schwefelatom steht,

$R^2$ — für Mercapto, Hydroxy, geradkettiges oder verzweigtes Alkoxy mit bis zu 5 Kohlenstoffatomen oder für die Gruppe der Formel

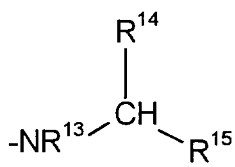

steht,
worin

$R^{13}$ — Wasserstoff oder geradkettiges oder verzweigtes Alkyl mit bis zu 3 Kohlenstoffatomen bedeutet,

$R^{14}$ — Wasserstoff, Phenyl, Pyridyl oder Thienyl bedeutet,

$R^{15}$ — Wasserstoff oder geradkettiges oder verzweigtes Alkyl mit bis zu 3 Kohlenstoffatomen bedeutet, das gegebenenfalls durch Hydroxy substituiert ist,

und deren Salze.

Ganz besonders bevorzugt sind Verbindungen der allgemeinen Formel (I), in welcher

A — für einen Rest der Formel

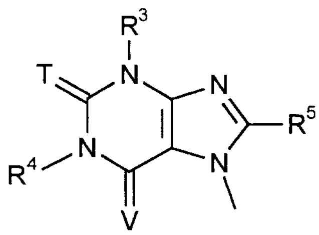

oder

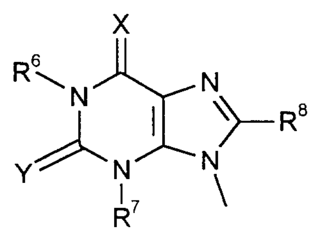

steht,
worin

$R^3$, $R^4$, $R^6$ und $R^7$ — Wasserstoff, geradkettiges oder verzweigtes Alkyl mit jeweils bis zu 4 Kohlenstoffatomen bedeuten,

T, V, X und Y — gleich oder verschieden sind und ein Sauerstoff- oder Schwefelatom bedeuten,

$R^5$ und $R^8$ — gleich oder verschieden sind und Wasserstoff, Fluor, Chlor, Brom, Cyclopropyl, Cyclopentyl, Cyclohexyl oder für geradkettiges oder verzweigtes Alkyl oder Alkenyl mit jeweils bis zu 3 Kohlenstoffatomen bedeuten, die gegebenenfalls durch Cyclopropyl, Cyclopentyl, Cyclohexyl, Phenyl, Pyridyl, Thienyl oder Furyl substituiert sind, die ihrerseits bis zu 2-fach gleich oder verschieden durch Phenyl, Benzyl, Fluor, Chlor, Brom, Hydroxy oder durch geradkettiges oder verzweigtes Alkyl oder Alkoxy mit jeweils bis zu 3 Kohlenstoffatomen substituiert sein können,
oder
Phenyl, Pyridyl, Thienyl oder Furyl bedeuten, die gegebenenfalls bis zu 2-fach gleich oder verschieden durch Fluor, Chlor, Brom, Phenyl, Trifluormethyl, Hydroxy oder durch

geradkettiges oder verzweigtes Alkyl oder Alkoxy mit jeweils bis zu 3 Kohlenstoffatomen oder durch eine Gruppe der Formel $-(CO)_a-NR^9R^{10}$ substituiert sind, worin

| | |
|---|---|
| a | die Zahl 0 bedeutet, |
| $R^9$ und $R^{10}$ | gleich oder verschieden sind und Wasserstoff, oder geradkettiges oder verzweigtes Alkyl mit jeweils bis zu 3 Kohlenstoffatomen bedeuten, |
| D und E | gleich oder verschieden sind und für Wasserstoff, Fluor, Chlor, Brom oder Trifluormethyl stehen, |
| $R^1$ | für Wasserstoff, Cyclopropyl, Cyclopentyl, Cyclohexyl, Cycloheptyl, Phenyl oder für geradkettiges oder verzweigtes Alkyl mit bis zu 5 Kohlenstoffatomen steht, |
| L | für ein Sauerstoffatom steht, |
| $R^2$ | für Hydroxy, geradkettiges oder verzweigtes Alkoxy mit bis zu 5 Kohlenstoffatomen oder für die Gruppe der Formel 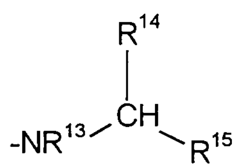 steht, worin |
| $R^{13}$ | Wasserstoff oder geradkettiges oder verzweigtes Alkyl mit bis zu 3 Kohlenstoffatomen bedeutet, |
| $R^{14}$ | Phenyl bedeutet, |
| $R^{15}$ | geradkettiges oder verzweigtes Alkyl mit bis zu 3 Kohlenstoffatomen bedeutet, das gegebenenfalls durch Hydroxy substituiert ist, |

und deren Salze.

Außerdem wurde ein Verfahren zur Herstellung der erfindungsgemäßen Verbindungen der allgemeinen Formel (I) gefunden, dadurch gekennzeichnet, daß man zunächst durch Umsetzung von Verbindungen der allgemeinen Formel (II)

$$A\text{-}H \qquad (II)$$

in welcher

A die oben angegebene Bedeutung hat,

mit Verbindungen der allgemeinen Formel (III)

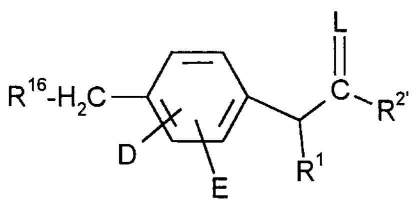

(III)

in welcher

D, E , L und $R^1$ die oben angegebene Bedeutung haben,

$R^{16}$ für Hydroxy oder Halogen, vorzugsweise für Brom steht,

und

$R^{2'}$ für geradkettiges oder verzweigtes Alkoxy mit bis zu 8 Kohlenstoffatomen steht,

in inerten Lösemitteln und in Anwesenheit von Basen und/oder Hilfsmitteln in Verbindungen der allgemeinen Formel (Ia)

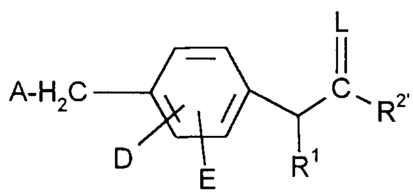

(Ia)

in welcher

A, D, E, L, $R^1$ und $R^{2'}$ die oben angegebene Bedeutung haben,

überführt und diese gegebenenfalls ($R^2$ = OH) verseift,
oder gegebenenfalls diese Säuren mit Glycinolen und Glycinolderivaten der allgemeinen Formel (IV)

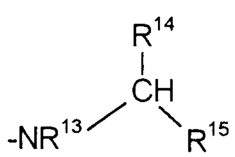

(IV)

in welcher

$R^{13}$, $R^{14}$ und $R^{15}$ die oben angegebene Bedeutung haben,

in inerten Lösemitteln, gegebenenfalls in Anwesenheit von Basen und/oder Hilfsmitteln umsetzt.

Das erfindungsgemäße Verfahren kann durch folgendes Formelschema beispielhaft erläutert werden:

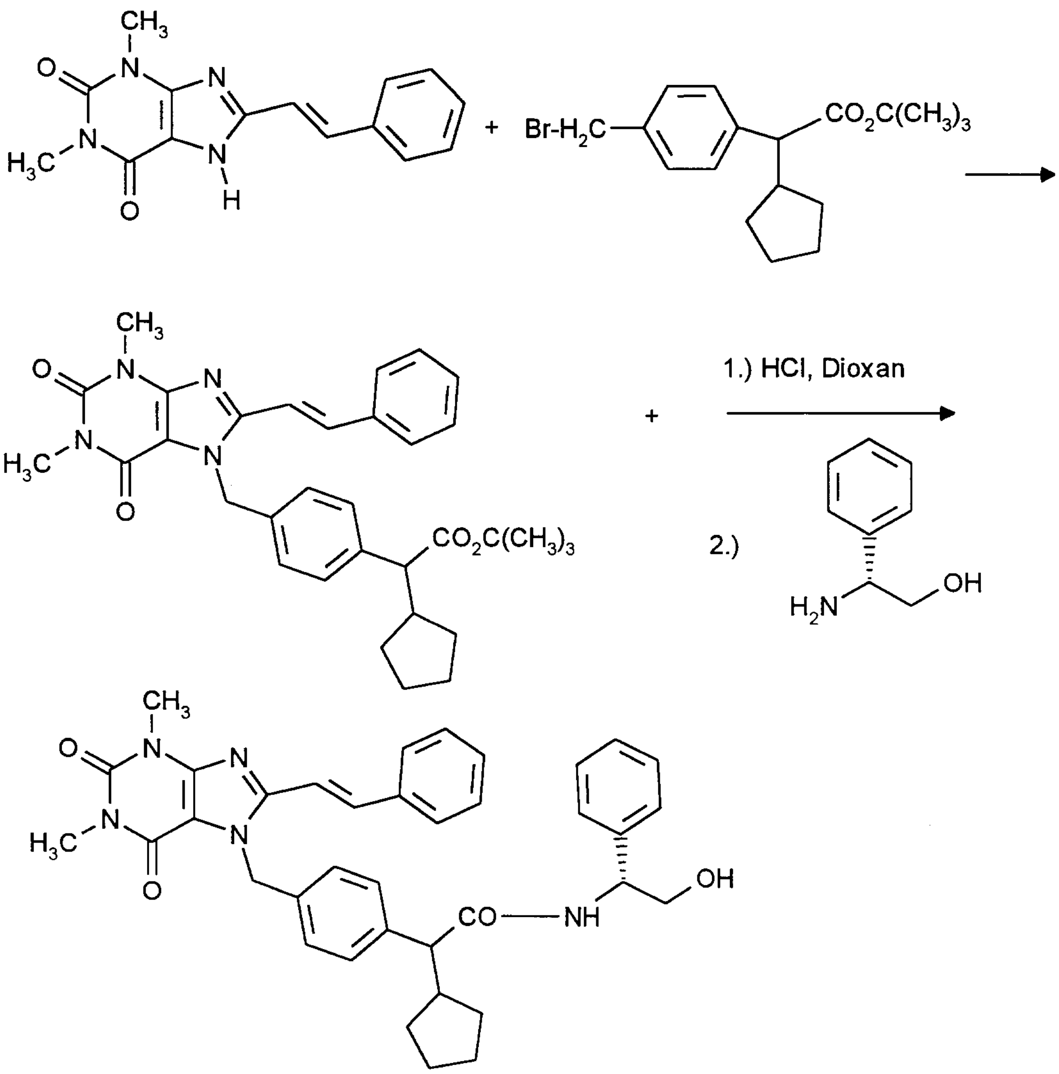


Für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Alkohole wie beispielsweise Methanol, Ethanol, Propanol, Isopropanol, Butanol, iso-Butanol oder tert. Butanol, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat, DBU oder DABCO.

Im allgemeinen setzt man die Base in einer Menge von 0,05 mol bis 10 mol, bevorzugt von 1 mol bis 2 mol, bezogen auf 1 mol der Verbindung der Formel (II) ein.

Die erfindungsgemäßen Verfahren werden im allgemeinen in einem Temperaturbereich von -30°C bis +100°C, bevorzugt von -10°C bis +60°C, durchgeführt.

Die erfindungsgemäßen Verfahren werden im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt wird Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Als Lösemittel für die Umsetzung mit Glycinolen eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohenwasserstoffe wie Benzol, Xylol, Toluol, Hexan, Cyclohexan oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt ist Dichlormethan, Tetrahydrofuran, Aceton oder Dimethylformamid.

Als Basen können hier im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat und Triethylamin.

Die Base wird in einer Menge von 1 mol bis 5 mol, bevorzugt von 1 mol bis 3 mol, bezogen auf 1 mol der entsprechenden Carbonsäure eingesetzt.

Die Reaktion wird im allgemeinen in einem Temperaturbereich von 0°C bis 150°C, bevorzugt von + 20°C bis + 110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Die Umsetzung mit Phenylglycinolen kann gegebenenfalls auch über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die oben aufgeführten Basen können gegebenenfalls auch als säurebindende Hilfsmittel eingesetzt werden.

Als Hilfsmittel eignen sich ebenso Dehydratisierungsreagenzien. Dazu gehören beispielsweise Carbodiimide wie Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Iso-butylchloroformat oder Benzotriazolyloxy-tris-(dimethylamino)phosphoniumhexa-fluorophosphat oder Phosphorsäurediphenyl-esteramid oder Methan-sulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formeln (III) und (IV) sind an sich bekannt oder nach üblichen Methoden herstellbar.

Die Verbindungen der allgemeinen Formel (II) sind bekannt oder können hergestellt werden, indem man beispielsweise die entsprechenden 1,3-substituierten Uracile mit Orthoameisensäureester, Carbonsäurechloriden und mit Aldehyden der allgemeinen Formel (V)

$$R^{17}\text{-CHO} \qquad (V)$$

in welcher

$R^{17}$ die oben angegebene Bedeutungen von $R^5$ und/oder $R^8$ umfaßt,

in inerten Lösemitteln und in Anwesenheit einer Base umsetzt.

Für die Herstellung eignen sich die oben aufgeführten Lösemittel. Bevorzugt ist Ethanol, Dichlormethan oder Dimethylformamid.

Die Umsetzungen werden in einem Temperaturbereich von 30°C bis 160°C, vorzugsweise bei der Siedetemperatur des jeweiligen Lösemittels und Normaldurck durchgeführt.

Die Aldehyde der allgemeinen Formel (V) sind an sich bekannt oder nach üblichen Methoden herstellbar.

Die Verbindungen der allgemeinen Formel (Ia) sind neu und nach dem oben beschriebenen Verfahren herstellbar.

Die erfindungsgemäßen Verbindungen der allgemeinen Formeln (I) und (Ia) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von Koronaren Herzerkrankungen, Herzinsuffizienz, Störungen der Hirnleistung, ischämischen Gehirnerkrankungen, Apoplex, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

**1. Hemmung der Freisetzung ApoB-100-assoziierter Lipoproteine**

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtabsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtabsorption im Vergleich zur Kontrolle

(Lösemittelkontrolle ohne Substanz) um 50% inhibiert ist.

**Tabelle 1:**

| Bsp.-Nr. | ApoB $IC_{50}$ [nM] |
|---|---|
| 112 | 4.0 |
| 113 | 58.0 |
| 114 | 39.0 |
| 115 | 240.0 |
| 117 | 3.0 |
| 118 | 7.0 |
| 119 | 11.0 |
| 120 | 28.0 |
| 121 | 36.0 |
| 122 | 48.0 |
| 123 | 8.0 |
| 126 | 41.0 |
| 129 | 34.0 |
| 130 | 25.0 |
| 131 | 135.0 |
| 132 | 743.0 |
| 133 | 5.0 |
| 136 | 23.0 |
| 137 | 124.0 |
| 138 | 403.0 |
| 139 | 90.0 |
| 140 | 10.0 |
| 143 | 11.0 |
| 146 | 6.0 |
| 149 | 9.0 |
| 151 | 34.0 |
| 152 | 169.0 |
| 154 | 3.0 |
| 155 | 2.0 |
| 156 | 25.0 |

| Bsp.-Nr. | ApoB $IC_{50}$ [nM] |
|---|---|
| 157 | 8.0 |
| 158 | 2.0 |
| 159 | 25.0 |
| 160 | 5.0 |
| 163 | 131.0 |
| 164 | 12.0 |
| 167 | 19.0 |
| 168 | 16.0 |
| 169 | 3.0 |
| 170 | 16.0 |
| 171 | 2.0 |
| 172 | 1.0 |
| 174 | 5.0 |
| 175 | 32.0 |
| 176 | 19.0 |
| 178 | 64.0 |
| 179 | 5.0 |
| 180 | 5.0 |
| 181 | 10.0 |
| 182 | 12.0 |
| 183 | 10.0 |
| 184 | 24.0 |
| 185 | 29.0 |
| 188 | 23.0 |
| 189 | 31.0 |
| 190 | 86.0 |
| 191 | 14.0 |
| 192 | 8.0 |
| 193 | 62.0 |
| 194 | 8.0 |
| 195 | 5.0 |
| 196 | 61.0 |

| Bsp.-Nr. | ApoB $IC_{50}$ [nM] |
|---|---|
| 197 | 43.0 |
| 198 | 5.0 |
| 199 | 5.0 |
| 200 | 24.0 |
| 201 | 11.0 |
| 202 | 9.0 |
| 203 | 63.0 |
| 204 | 16.0 |
| 207 | 40.0 |
| 211 | 9.0 |
| 212 | 8.0 |
| 215 | 3.0 |
| 216 | 34.0 |
| 217 | 11.0 |
| 218 | 8.0 |
| 219 | 5.0 |
| 220 | 16.0 |
| 223 | 4.0 |
| 224 | 4.0 |
| 225 | 4.0 |
| 226 | 11.0 |
| 227 | 40.0 |

**2. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in

diesem Modell entweder unmittelbar vor Applikation des Detergens intravenös verabreicht oder vor Einleitung der Narkose oral oder subkutan.

Tabelle 2

| Bsp.-Nr. | Triton-Hamster %TG Inhibition 2 h nach Substanz | Triton-Hamster %CH Inhibition 2 h nach Substanz |
|---|---|---|
| 129 | 25% bei 6 mg/kg i.v. | 25% bei 6 mg/kg i.v. |

**3. Hemmung der intestinalen Triglyceridabsoprtion in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus entnommen. Anschließend werden die Traganth-Suspension, die Traganth-OlivenölSuspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Triglyceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\%\ \text{Triglyceridanstieg} = \frac{\Delta TG_{\text{Substanz}} - \Delta TG_{\text{Traganthkontrolle}}}{\Delta TG_{\text{Ölbelastung}} - \Delta TG_{\text{Traganthkontrolle}}} \times 100$$

Effekt von 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Tabelle 3

| Bsp.-Nr. | Absorption $ED_{50}$ oder % Inhibition (mg/kg p.o.) |
|---|---|
| 112 | 3 mg/kg |
| 114 | < 2 mg/kg |
| 117 | 3 mg/kg |
| 123 | 2 mg/kg |
| 129 | 20 mg/kg |
| 130 | 5 mg/kg |
| 133 | 2 mg/kg |
| 136 | 2 mg/kg |
| 140 | > 3 mg/kg |
| 143 | > 3 mg/kg |
| 146 | > 3 mg/kg |
| 149 | > 3 mg/kg |
| 151 | 6 mg/kg |
| 154 | < 2 mg/kg |
| 157 | > 2 mg/kg |
| 160 | 3 mg/kg |
| 167 | > 2 mg/kg |
| 169 | 2 mg/kg |
| 174 | < 2 mg/kg |
| 178 | 3 mg/kg |
| 179 | > 3 mg/kg |
| 182 | 2 mg/kg |
| 191 | >> 3 mg/kg |
| 197 | 3 mg/kg |
| 201 | > 3 mg/kg |
| 227 | > 6 mg/kg |

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0,05$) um mindestens 30 % vermindern, werden als pharmakologisch wirksam angesehen.

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht (2,5 ml/kg) Triton WR-1339, gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholsterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Test-substanzen werden unmittelbar nach der Tritoninjektion oral, intravenös oder subcutan appliziert.

Tabelle 4

| Bsp.-Nr. | Triton Ratte % TG Inhibition (p.o.) 2 h nach Substanz |
|---|---|
| 112 | 17.0% bei 3 mg/kg (n.s.) |
| 114 | 14.1% bei 3 mg/kg (n.s.) |
| 117 | 25% bei 3 mg/kg (n.s.) |
| 130 | 36% bei 20 mg/kg |
| 136 | 28% bei 3 mg/kg |
| 154 | (-4%) bei 3 mg/kg (n.s.) |
| 157 | 11.3% bei 3 mg/kg |
| 169 | 59.4% bei 10 mg/kg |
| 169 | 41 % bei 5 mg/kg |
| 169 | 28% bei 3 mg/kg |
| 175 | 9.8% bei 3 mg/kg (n.s.) |
| 176 | 6.2% bei 3 mg/kg (n.s.) |
| 227 | (-8%) bei 3 mg/kg (n.s.) |

Die Erfindung betrifft außerdem die Kombination von substituierten Xanthinen der allgemeinen Formel (I) mit einem Glucosidase- und/oder Amylasehemmer zur Behandlung von familiärer Hyperlipidamien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose, MDL-73945, Tendamistat, AI-3688, Trestatin, Pradimilin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1

mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Verwendete Abkürzungen:**

| | |
|---|---|
| Ac | = Acetyl |
| bs | = broad singlet |
| Bn | = Benzyl |
| Bz | = Benzoyl |
| CI | = Chemische Ionisation |
| cDec | = cyclo Decyl |
| cDodec | = cyclo Dodecyl |
| cHept | = cyclo Heptyl |
| cHex | = cyclo Hexyl |
| cNon | = cyclo Nonyl |
| cOct | = cyclo Octyl |
| cPent | = cyclo Pentyl |
| cPr | = cyclo Propyl |
| cUndec | = cyclo Undecyl |
| d | = doublet |
| DCC | = Dicyclohexylcarbodiimid |
| DCCI | = N'-(3-Dimethylaminopropyl)-N'-ethylcarbodiimid |
| dd | = doublet doublets |
| DDQ | = 2,3-Dichlor-5,6-dicyano-1,4-benzochinon |
| dia | = Diastereomer |
| DMAP | = 4-(N,N-Dimethylamino)pyridin |
| DMF | = N,N-Dimethylformamid |
| DMSO | = Dimethylsulfoxid |
| EI | = Elektronenstoß-Ionisation |
| ent | = Enantiomer |
| Et | = Ethyl |
| FAB | = Fast Atom Bombardment |
| HOBT | = 1-Hydroxy-1H-benzotriazol |
| Hz | = Hertz |
| iBu | = iso Butyl |
| iPr | = iso Propyl |
| m | = multiplet |
| Me | = Methyl |
| Mes | = Mesyl |
| NBS | = Bromsuccinimid |
| nBu | = normales Benzyl |
| nPr | = normales Propyl |
| Ph | = Phenyl |
| PPA | = Polyphosphorsäure |
| pTol | = para Tolyl |
| pTos | = para Tosyl |
| rac | = Racemat |
| RT | = Raumtemperatur |
| s | = singlet |
| sBu | = sekundär Butyl |
| tBu | = tertiär Butyl |
| t | = triplet |
| TFA | = Trifluoressigsäure |

THF = Tetrahydrofuran
TMS = Tetramethylsilan

| Benutzte Solvenzgemische | |
|---|---|
| Petrolether : Aceton | = 1:1 (A) |
| Petrolether : Essigester | = 20:1 (B) |
| Petrolether : Essigester | = 10:1 (C) |
| Petrolether : Essigester | = 5:1 (D) |
| Petrolether : Essigester | = 3:1 (E) |
| Petrolether : Essigester | = 2:1 (F) |
| Petrolether : Essigester | = 1:1 (G) |
| Petrolether : Essigester | = 1:2 (H) |
| Dichlormethan (100%) | = (I) |
| Dichlormethan : Methanol | = 50:1 (J) |
| Dichlormethan : Methanol | = 20:1 (K) |
| Dichlormethan : Methanol | = 10:1 (L) |
| Dichlormethan : Essigester | = 1:1 (M) |
| Dichlormethan : Ethanol | = 50:1 (N) |
| Essigester : Methanol | = 10:1 (O) |
| Toluol (100%) | = (P) |
| Toluol : Essigester | = 1:1 (Q) |
| Toluol : Essigester | = 8:1 (R) |
| Toluol : Essigester | = 9:1 (S) |
| Petrolether : Essigester | = 4:1 (T) |

**Ergänzungen:**

Folgende Kennzeichnungen haben für alle nachfolgenen Tabellen Gültigkeit:
* = EI
# = CI

**Beispiel I** (Methode A)

1,3-Dimethyl-8-(4-methyl)phenyl-xanthin

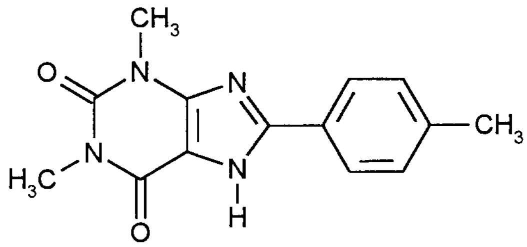

8.5 g (50 mmol) 5,6-Diamino-1,3-dimethyluracil Hydrat wurden unter Rückfluß in Ethanol (180 ml) gelöst. Zu diese Lösung wurde eine Lösung von 6.0 g (50 mmol) p-Tolylaldehyd sowie 4.5 g Essigsaeure in Ethanol (50 ml) gegeben. Es wurde für 1 Std unter Rückfluß erhitzt, auf Raumtemperatur abgekühlt und abgesaugt. Die Kristalle wurden mit Diethylether nachgewaschen.
Die so erhaltenen Kristalle wurden vorgelegt und mit 36.6 g (210 mmol) Azodicarbonsaeurediethylester versetzt. Es wurde 5 Min. auf 90°C erwärmt, wobei ein Feststoff ausfiel. Die Lösung wurde auf Raumtemperatur abgekühlt und mit Ethanol (100 ml) verdünnt. Der ausgefallene Feststoff wurde abgesaugt, mit Diethylether gewaschen und i. Vak. getrocknet.
Ausbeute 10.9 g (81%);
$R_f$ = 0.56 (Dichlormethan : Methanol, 20 : 1);
Fp = >240 °C;
Masse (berechnet) für $C_{14}H_{14}N_4O_2$ = 270.30, Massenspektrum (EI, rel. Intensität) 270 (100%);
$^1H$ NMR (200 MHz, Pyridin-$D_5$) δ 8.29 (d, J = 8.62 Hz, 2 H), 7.33 (d, J = 7.89 Hz, 2 H), 4.98 (bs, 1 H), 3.72 (s, 3 H), 3.53 (s, 3 H), 2.29 (s, 3 H).

**Beispiel II** (Methode B)

1,3-Dimethyl-8-[1-(3-chlorphenyl)methyl]xanthin

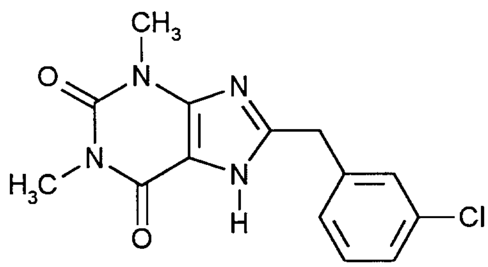

11.94 g (70 mmol) 3-Chlorphenylessigsäure wurden in 100 ml Dichlormethan gelöst, mit einem Tropfen DMF versetzt und auf 0°C gekühlt. Zu dieser Lösung gab man langsam 8.74 g (73.5 mmol) Thionylchlorid und rührte bis zum Ende der Gasentwicklung (ca. 1 h), wobei man das Reaktionsgemisch auf Raumtemperatur erwärmen ließ.
11.91 g (70 mmol) 5,6-Diamino-1,3-dimethyluracil Hydrat wurden in 1M NaOH (150 ml) und Wasser (350 ml) vorgelegt und auf 45°C erwärmt bis eine homogene Lösung entstand. Man kühlte diese Lösung auf Raumtemperatur ab und gab die Säurechloridlösung (s.o.) unter kräftigem Rühren hinzu. Es wurden noch weitere 16 h bei Raumtemperatur gerührt. Der ausgefallene Feststoff wurde abgesaugt und mit Wasser gewaschen.
Dieser Feststoff wurde in Methanol (400 ml) suspendiert und mit 4 M NaOH (400 ml) 1 h unter Rückfluß erhitzt. Nach Abkühlen auf Raumtemperatur säuerte man die Lösung mit konz. HCl auf pH 3 an, wobei das gewünschte Produkt ausfiel. Der ausgefallene Feststoff wurde abgesaugt, mit Wasser und Methanol gewaschen und im Umlufttrockenschrank getrocknet.

Ausbeute 17.1 g (80%);
$R_f$ = 0.57 (Dichlormethan : Methanol, 10 : 1);
Fp = >240 °C;
Masse (berechnet) für $C_{14}H_{13}ClN_4O_2$ = 304.74, Massenspektrum (EI, rel. Intensität) 304 (100%);
$^1H$ NMR (250 MHz, Trifluoressigsäure-$D_1$) δ 7.36-7.30 (m, 3 H), 7.20 (m, 1 H), 4.50 (s, 2 H), 3.74 (s, 3 H), 3.52 (s, 3 H).

**Beispiel III** (Methode C)

1,3-Diethylxanthin

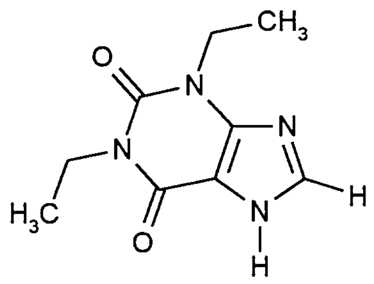

5.0 g (25 mmol) 5,6-Diamino-1,3-diethyluracil und 475 mg (2.5 mmol) p-Toluolsulfonsaeure wurden in Orthoameisensaeuretrimethylester (50 ml) gelöst. Es wurde für 1 Std. zum Rückfluß gekocht, dann abgekühlt und abgesaugt. Da eine polare Verunreinigung enstanden war, wurde die Substanz durch Chromatographie an Kieselgel gereinigt:
Ausbeute 3.5 g (67%);
$R_f$ = 0.15 (Dichlormethan : Methanol, 20 : 1);
Fp = 218-220 °C;
Masse (berechnet) für $C_9H_{12}N_4O_2$ = 208.23, Massenspektrum (EI, rel. Intensität) 208 (100%);
$^1H$ NMR (200 MHz, DMSO-$D_6$) δ 13.55 (bs, 1 H), 8.05 (s, 1 H), 4.04 (q, J = 7.07 Hz, 2 H), 3.93 (q, J = 7.02 Hz, 2 H), 1.23 (t, J = 7.03 Hz, 3 H), 1.13 (t, J = 7.01 Hz, 3 H).

Die in der Tabelle I aufgeführten Verbindungen wurden nach den dort aufgeführten Methoden hergestellt:

**Tabelle I:**

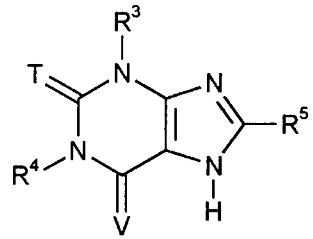

| Bsp.-Nr. | $R^4$ | $R^3$ | $R^5$ | T | V | $R_f$ * | F (°C) | $CH_3$thode | Massen-spektrum | Ausbeute (% d.Th.) |
|---|---|---|---|---|---|---|---|---|---|---|
| IV | $CH_3$ | $CH_3$ | $CH_3$ | O | O | 0.57 (L) | >240 | C | *194 (100%) | 50 |
| V | $CH_3$ | $CH_3$ | $C_2H_5$ | O | O | 0.56 (K) | >240 | C | *208 (100%) | 46 |
| VI | $CH_3$ | $CH_3$ | cPro | O | O | 0.35 (K) | >240 | A | *220 (100%) | 47 |
| VII | $CH_3$ | $CH_3$ | $-CH_2$cHex | O | O | 0.64 (L) | 238 | B | *276 (40%) 194 (100%) | 72 |
| VIII | $CH_3$ | $CH_3$ | 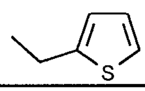 | O | O | 0.80 (L) | >240 | B | 276 (100%) | 47 |
| IX | $CH_3$ | $CH_3$ | 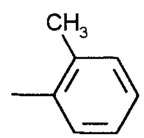 | O | O | 0.67 (K) | >240 | A | *270 (100%) | 82 |
| X | $CH_3$ | $CH_3$ | 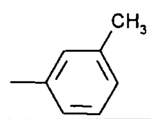 | O | O | 0.86 (L) | >240 | A | *270 (100%) | 79 |
| XI | $CH_3$ | $CH_3$ | 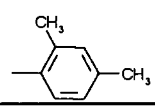 | O | O | 0.80 (L) | >240 | A | *284 (100%) | 69 |

| Bsp.-Nr. | $R^4$ | $R^3$ | $R^5$ | T | V | $R_f$ * | F (°C) | $CH_3$thode | Massen-spektrum | Ausbeute (% d.Th.) |
|---|---|---|---|---|---|---|---|---|---|---|
| XII | $CH_3$ | $CH_3$ | 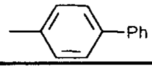 | O | O | 0.85 (L) | >240 | B | *332 (100%) | 73 |
| XIII | $CH_3$ | $CH_3$ | 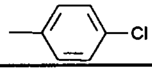 | O | O | 0.77 (L) | >240 | A | *290 (100%) | 73 |
| XIV | $CH_3$ | $CH_3$ | 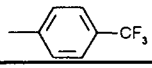 | O | O | 0.75 (L) | >240 | A | *324 (100%) | 69 |
| XV | $CH_3$ | $CH_3$ | 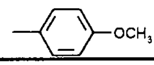 | O | O | 0.80 (L) | >240 | A | *286 (100%) | 76 |
| XVI | $CH_3$ | $CH_3$ | 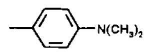 | O | O | --- | >240 | A | *299 (100%) | 77 |
| XVII | $CH_3$ | $CH_3$ | 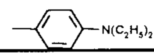 | O | O | | >250 | A | #328 (100%) | 81 |
| XVIII | $CH_3$ | $CH_3$ | 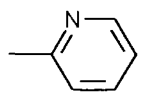 | O | O | 0.33 (K) | >240 | A | 257 (100%) | 58 |
| XIX | $CH_3$ | $CH_3$ | 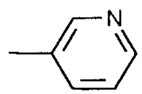 | O | O | 0.26 (K) | >240 | A | *257 (100%) | 66 |
| XX | $CH_3$ | $CH_3$ | 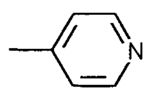 | O | O | 0.15 (K) | >240 | A | *258 (100%) | 21 |
| XXI | $CH_3$ | $CH_3$ | 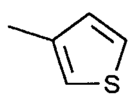 | O | O | 0.40 (K) | >240 | A | *262 (100%) | 80 |

| Bsp.-Nr. | $R^4$ | $R^3$ | $R^5$ | T | V | $R_f$ * | F (°C) | $CH_3$thode | Massen-spektrum | Ausbeute (% d.Th.) |
|---|---|---|---|---|---|---|---|---|---|---|
| XXII | $CH_3$ | $CH_3$ | 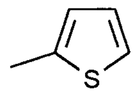 | O | O | | >240 | A | | 88 |
| XXIII | $CH_3$ | $CH_3$ | 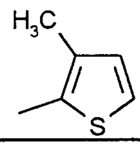 | O | O | 0.30 (K) | >240 | A | 276 (100%) | 78 |
| XXIV | $CH_3$ | $CH_3$ | 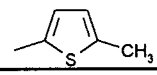 | O | O | 0.38 (K) | >240 | A | 276 (100%) | 75 |
| XXV | $CH_3$ | $CH_3$ | 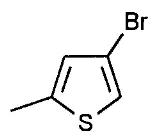 | O | O | 0.54 (K) | >240 | A | 341 (100%) | 83 |
| XXVI | $CH_3$ | $CH_3$ | 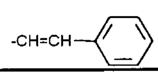 | O | O | 0.69 (K) | >240 | A | 283 (100%) | 70 |
| XXVII | $CH_3$ | $CH_3$ | 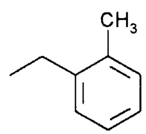 | O | O | 0.68 (L) | >240 | B | *284 (100%) | 68 |
| XXVIII | $CH_3$ | $CH_3$ | 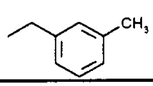 | O | O | 0.68 (L) | >240 | B | *284 (100%) | 64 |
| XXIX | $CH_3$ | $CH_3$ | 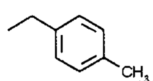 | O | O | 0.28 (K) | >240 | B | *284 (100%) | 64 |
| XXX | $CH_3$ | $CH_3$ | 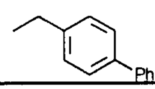 | O | O | 0.78 (L) | >240 | B | *346 (100%) | 47 |

| Bsp.-Nr. | $R^4$ | $R^3$ | $R^5$ | T | V | $R_f$ * | F (°C) | $CH_3$thode | Massen-spektrum | Ausbeute (% d.Th.) |
|---|---|---|---|---|---|---|---|---|---|---|
| XXXI | $CH_3$ | $CH_3$ | 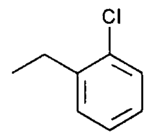 | O | O | 0.57 (L) | >240 | B | *304 (100%) | 71 |
| XXXII | $CH_3$ | $CH_3$ | 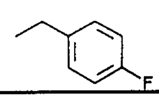 | O | O | 0.64 (L) | >240 | B | *288 (100%) | 49 |
| XXXIII | $CH_3$ | $CH_3$ | 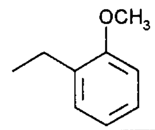 | O | O | 0.61 (L) | >240 | B | *300 (100%) | 64 |
| XXXIV | $CH_3$ | $CH_3$ | 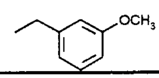 | O | O | 0.64 (K) | >240 | B | *300 (100%) | 66 |
| XXXV | $CH_3$ | $CH_3$ | 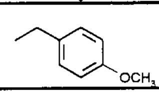 | O | O | 0.27 (K) | >240 | B | *300 (100%) | 23 |
| XXXVI | $CH_3$ | $CH_3$ | 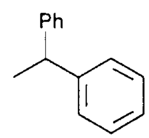 | O | O | 0.74 (K) | 143-45 | A | *346 (100%) | 7 |
| XXXVII | $C_2H_5$ | $C_2H_5$ | $CH_3$ | O | O | 0.83 (K) | 231-33 | C | *222 (100%) | 54 |
| XXXVIII | $C_2H_5$ | $C_2H_5$ | $C_2H_5$ | O | O | 0.71 (K) | 193-94 | C | *236 (100%) | 67 |
| XXXIX | $C_2H_5$ | $C_2H_5$ | cPro | O | O | 0.69 (L) | 217-18 | A | #249 (100%) | 39 |
| XL | $C_2H_5$ | $C_2H_5$ | 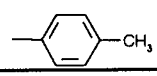 | O | O | 0.76 (L) | >240 | A | #299 (100%) | 70 |
| XLI | $C_2H_5$ | $C_2H_5$ | 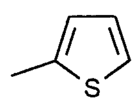 | O | O | 0.67 (L) | >240 | A | #291 (100%) | 82 |

| Bsp.-Nr. | $R^4$ | $R^3$ | $R^5$ | T | V | $R_f$ * | F (°C) | $CH_3$thode | Massen-spektrum | Ausbeute (% d.Th.) |
|---|---|---|---|---|---|---|---|---|---|---|
| XLII | $CH_3$ | $CH_3$ | $CH_3$ | S | O | 0.30 (K) | >240 | C | *210 (100%) | 51 |
| XLIII | $CH_3$ | $CH_3$ | $C_2H_5$ | S | O | 0.62 (K) | >240 | C | *224 (100%) | 78 |
| XLIV | $CH_3$ | $CH_3$ | 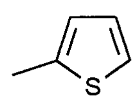 | S | O | 0.79 (K) | >240 | A | *278 (100%) | 51 |
| XLV | $CH_3$ | $CH_3$ | 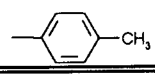 | S | O | 0.80 (K) | >240 | A | *286 (100%) | 59 |

**Beispiel XLVI**

2-(R&S)-Phenyl-2-(4-methyl)phenylessigsäuremethylester

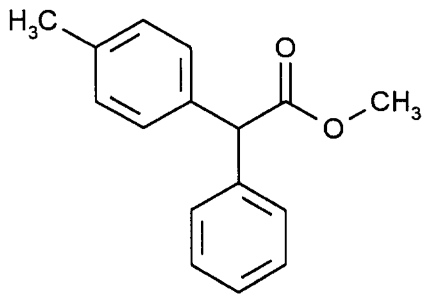

21.0 g (100 mmol, Apin) 2-Phenyl-1-(4-methyl)phenyl-1-oxoethane und 38.8 g (120 mmol) Iodbenzoldiacetat wurden in 300 ml Orthoameisensaeuretrimethylester gelöst. Zu dieser Lösung wurden 19.6 g konz. Schwefelsäure gegeben, und die Lösung wurde für 6 Std auf 60°C erwärmt. Die Lösung wurde auf Raumtemperatur abgekühlt, mit Wasser (100 ml) verdünnt, und mit Diethylether extrahiert. Die vereinigten organischen Phasen wurden über Natriumsulfat getrocknet und einrotiert. Der Rückstand wurde säulenchromatographisch gereinigt.
Ausbeute 13.1 g (55%);
$R_f$ = 0.33 (Petrolether : Essigester, 20 : 1);
Masse (berechnet) für $C_{16}H_{16}O_2$ = 240.30, Massenspektrum (FAB, rel. Intensität) 241 (25%), 181 (100%);
$^1H$ NMR (200 MHz, $CDCl_3$) δ 7.3-7.10 (m, 9 H), 4.99 (s, 1 H), 3.73 (s, 3 H), 2.31 (s, 3 H).

**Beispiel XLVII**

2-Cyclopentyl-2-(4-methylphenyl)-essigsäure-tert.butylester

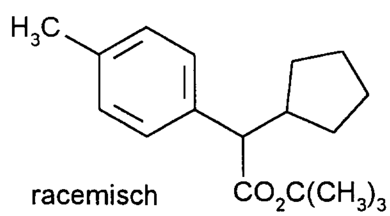

racemisch

33.5 g (0.3 mol) Kalium-tert.butylat werden in 100 ml wasserfreiem DMF bei 0°C vorgelegt, und 51.6 g (0.25 mol) 4-Methylphenyl-essigsäure-tert.butylester in 250 ml wasserfreiem DMF zugetropft. Es wird 30 min. bei 0°C gerührt und 32.2 ml (0.3 mol) Cyclopentylbromid in 150 ml wasserfreiem DMF bei 5-15°C zugetropft und 20 h bei 25°C gerührt. Nach Einengen wird der Rückstand zwischen Wasser und Diethylether verteilt, die Etherphase über Natriumsulfat getrocknet und eingeengt. Das Produkt kristallisiert aus.
Ausbeute: 67 g (97.5% d.Th.);
Festpunkt: 51-53°C.

Die Verbindungen der Tabelle II werden analog der Vorschrift des Beispiels XLVII hergestellt:

**Tabelle II:**

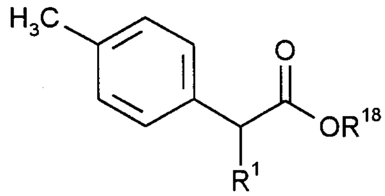

| Bsp.-Nr. | $R^1$ | $R^{18}$ | $R_f$ * |
|---|---|---|---|
| XLIII | (R&S) iPr | $CH_3$ | 0.86 (S) |
| IL | (R&S) iBu | tBu | 0.84 (R) |
| L | (R&S) cPent | $CH_3$ | 0.59 (C) |
| LI | (R&S) cHex | $CH_3$ | 0.38 (B) |
| LII | (R&S) cHex | tBu | 0.71 (P) |
| LIII | (R&S) cHept | $CH_3$ | 0.57 (P) |
| LIV | (R&S) cHept | tBu | 0.32 (P) |

**Beispiel LV**

2-(4-Brommethyl-phenyl)-2-cyclopentyl-essigsäure-tert.butylester

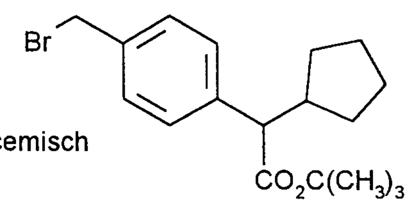

racemisch

27.4 g (0.1 mol) der Verbindung aus Beispiel XLVII werden in 200 ml Tetrachlormethan gelöst und zum Sieden erhitzt. Nach Zugabe von 0.82 g Azobisisobutyronitril werden 18.7 g (0.105 mol) N-Bromsuccinimid portionsweise zugegeben und anschließend 1 h refluxiert, auf 0°C abgekühlt und vom Succinimid abfiltriert. Nach Einengen des Filtrats fällt das Produkt aus. Es wird mit Petrolether (40/60) gewaschen und getrocknet.

Ausbeute: 20 g (57% d.Th.);

Fp.: 73-76°C.

Die Verbindungen der Tabelle III werden analog der Vorschrift des Beispiels LV hergestellt:

## Tabelle III

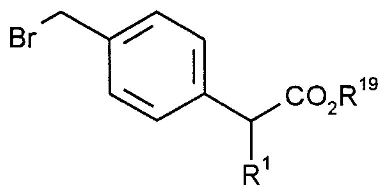

| Bsp.-Nr. | $R^1$ | $R^{19}$ | $R_f$ * | Ausgangsverbindung |
|---|---|---|---|---|
| LVI | (R&S) iPr | $CH_3$ | 0.78 (I) | XLIII |
| LVII | (R&S) iBu | tBu | 0.86 (I) | IL |
| LVIII | (R&S) cPent | $CH_3$ | 0.63 (C) | L |
| LIX | (R&S) cHex | $CH_3$ | 0.74 (I) | LI |
| LX | (R&S) cHex | tBu | 0.58 (C) | LII |
| LXI | (R&S) cHept | $CH_3$ | 0.59 (P) | LIII |
| LXII | (R&S) cHept | tBu | 0.84 (I) | LIV |
| LXIII | (R&S) Ph | $CH_3$ | 0.74 (I) | XLVI |

## Herstellungsbeispiele

### Beispiel 1

2-(R& S)-Cyclopentyl-2-[4-(1,3-dimethyl-2,6-dioxo-8-(4-methyl)phenyl-1,2,3,6-tetrahydro-purin-7-ylmethyl)phenyl]essigsäure-tert.Butylester

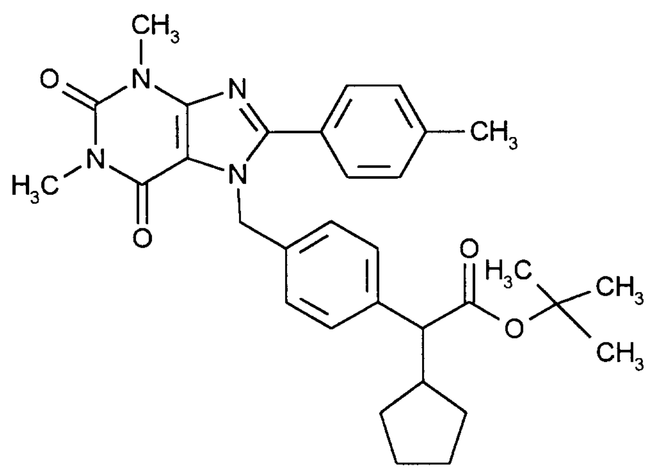

5.4 g (25 mmol) der Verbindung aus Beispiel I wurden bei 22°C in DMF (100 ml) suspendiert und mit 0.8 g (60% in Paraffin, 22 mmol) NaH versetzt. Nach 30 min bei 40 °C, wurde die Lösung mit der Verbindung aus Beispiel LV versetzt. Es wurde noch weitere 16 h bei Raumtemperatur gerührt. Wasser wurde dazugegeben und der anfallende Niederschlag abgesaugt. Der mäßig feuchte Feststoff wurde mit Dichlormethan verrührt und abgesaugt.

Ausbeute 9.3 g (88%);
$R_f$ = 0.48 (Dichlormethan : Methanol, 20 : 1);
Masse (berechnet) für $C_{32}H_{38}N_4O_4$ = 542.68, Massenspektrum (CI ($NH_3$), rel. Intensität) 560 (25%, M+$NH_4$), 543 (100%);
$^1H$ NMR (300 MHz, $CDCl_3$) δ 7.42 (d, 2 H), 7.28-7.20 (m, 4 H), 6.95 (d, 2 H), 5.60 (s, 2 H), 3.61 (s, 3 H), 3.40 (s, 3 H), 3.10 (d, 1 H), 2.42 (m, 1 H), 2.40 (s, 3 H), 1.90 (m, 1 H), 1.70-1.20 (m, 6 H), 1.41 (s, 9 H), 0.95 (m, 1 H).

Die in den Tabellen 1, 2 und 3 aufgeführten Verbindungen werden in Analogie zur Vorschrift des Beispiels 1 hergestellt:

Tabelle 1:

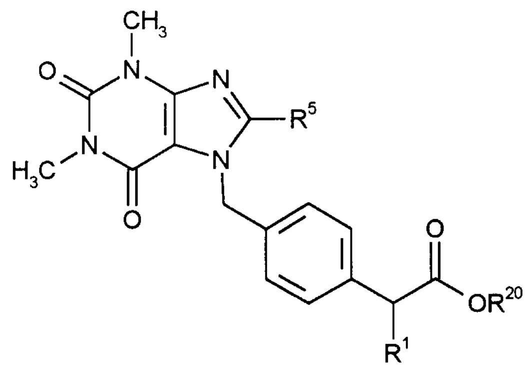

| Bsp.-Nr. | $R^1$ | $R^5$ | $R^{20}$ | $R_f$ * | F (°C) | Massen-spektrum | Ausbeute (% d.Th.) | Ausgangs-verbindung |
|---|---|---|---|---|---|---|---|---|
| 2 | (R&S) cPent | H | tBu | 0.54 (K) | 134 | 453 (100%) | 65 | |
| 3 | (R&S)cPent | Br | tBu | 0.40 (J) | 162 | 531 (25%)<br>57 (100%) | 74 | |
| 4 | (R&S)cPent | $CH_3$ | tBu | 0.23 (J) | 162 | 467 (100%) | 68 | IV |
| 5 | (R&S)cPent | Et | tBu | 0.22 (J) | 145 | 481 (40%)<br>57 (100%) | 50 | V |
| 6 | (R&S)cPent | cPr | tBu | 0.23 (J) | 74 | 493 (100%) | 70 | VI |
| 7 | (R&S)cPent | cPent | tBu | 0.30 (J) | 63 (Schaum) | 521 (20%)<br>57 (100%) | 68 | |
| 8 | (R&S)cPent | $-CH_2cHex$ | tBu | 0.38 (J) | 112 | 549 (80%)<br>57 (100%) | 71 | VII |
| 9 | (R&S)cPent | 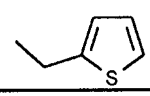 | $CH_3$ | 0.41 (J) | 148 | 508 (100%) | 66 | |

| Bsp.-Nr. | $R^1$ | $R^5$ | $R^{20}$ | $R_f$ * | F (°C) | Massen-spektrum | Ausbeute (% d.Th.) | Ausgangs-verbindung |
|---|---|---|---|---|---|---|---|---|
| 10 | (R&S)cPent | 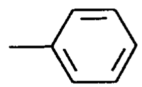 | tBu | 0.11 (T) | | | 84 | VIII |
| 11 | (R&S)cPent | 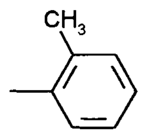 | tBu | 0.31 (J) | 105 | 543 (60%)<br>57 (100%) | 60 | IX |
| 12 | (R&S)cPent | 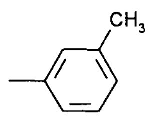 | tBu | 0.37 (J) | 98 | 543 (100%) | 64 | X |
| 13 | (R&S)cPent | 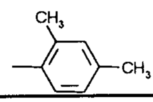 | tBu | 0.39 (J) | 88<br>(Schaum) | 557 (100%) | 66 | XI |
| 14 | (R&S)cPent | 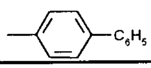 | $CH_3$ | 0.46 (J) | 104<br>(Schaum) | 563 (100%) | 52 | XII |
| 15 | (R&S)cPent | 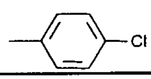 | tBu | 0.24 (J) | 90<br>(Schaum) | 563 (80%)<br>57 (100%) | 40 | XIII |
| 16 | (R&S)cPent | 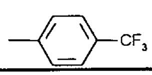 | tBu | 0.26 (J) | ---- | 597 (80%)<br>57 (100%) | 53 | XIV |
| 17 | (R&S)cPent | 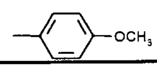 | tBu | 0.36 (J) | 96<br>(Schaum) | *559 (100%) | 51 | XV |
| 18 | (R&S)cPent | 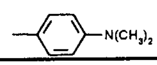 | tBu | 0.06 (J) | (Schaum) | 571 (80%)<br>298 (100%) | 36 | XVI |
| 19 | (R&S)cPent | 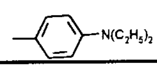 | tBu | 0.3 (J) | 84<br>(Schaum) | 600 (100%)<br>599 (100%) | 61 | XVII |

| Bsp.-Nr. | $R^1$ | $R^5$ | $R^{20}$ | $R_f$ * | F (°C) | Massen-spektrum | Ausbeute (% d.Th.) | Ausgangs-verbindung |
|---|---|---|---|---|---|---|---|---|
| 20 | (R&S)cPent | 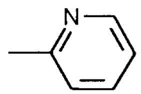 | tBu | 0.41 (J) | 155 | 529 (40%)<br>473 (100%) | 57 | XVIII |
| 21 | (R&S)cPent | 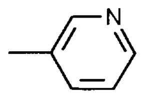 | tBu | 0.25 (J) | --- | 530 (50%)<br>57 (100%) | 20 | XIX |
| 22 | (R&S)cPent | 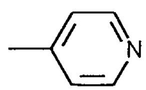 | tBu | 0.15 (K) | >240 | 530 (100%) | 52 | XX |
| 23 | (R&S)cPent | 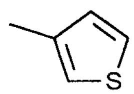 | tBu | 0.41 (J) | 173 | 535 (40%)<br>57 (100%) | 68 | XXI |
| 24 | (R&S)cPent | 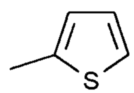 | tBu | 0.26 (E) | --- | 535 (100%)<br>534 (80%) | 60 | XXII |
| 25 | (R&S)cPent | 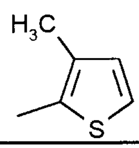 | tBu | 0.52 (J) | 193 | 549 (70%)<br>57 (100%) | 66 | XXIII |
| 26 | (R&S)cPent | 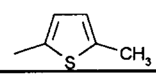 | tBu | 0.45 (J) | 78<br>(Schaum) | 549 (50%)<br>57 (100%) | 53 | XXIV |
| 27 | (R&S)cPent | 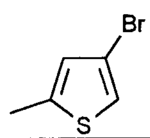 | tBu | 0.44 (J) | 165 | 615 (60%)<br>57 (100%) | 54 | XXV |
| 28 | (R&S)cPent | 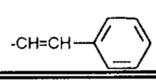 | tBu | 0.79 (G) | 207 | 555 (75%)<br>57 (100%) | 59 | XXVI |

Tabelle 2:

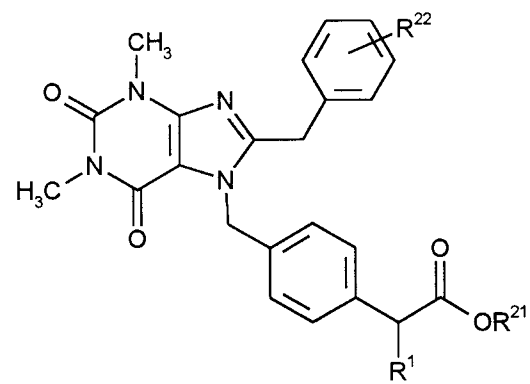

| Bsp.-Nr. | $R^1$ | $R^{21}$ | $R^{22}$ | $R_f$ * | F (°C) | Masssen-spektrum | Ausbeute (% d.Th.) | Ausgangs-verbindung |
|---|---|---|---|---|---|---|---|---|
| 29 | (R&S) cPent | tBu | H | 0.26 (J) | 136 | 543 (60%) 57 (100%) | 68 | |
| 30 | (R&S) cHex | $CH_3$ | H | 0.21 (J) | 131 | 515 (100%) | 80 | |
| 31 | (R&S) c Hept | tBu | H | 0.76 (K) | 82 | 571 (100%) | 86 | |
| 32 | (R&S) Phenyl | $CH_3$ | H | 0.23 (J) | 92 | 509 (100%) | 44 | |
| 33 | (R&S) cPent | tBu | 2-$CH_3$ | 0.29 (J) | 148 | 557 (100%) | 55 | XXVII |
| 34 | (R&S) cPent | tBu | 3-$CH_3$ | 0.40 (J) | 65 (Schaum) | 557 (60%) 57 (100%) | 51 | XXVIII |
| 35 | (R&S) cPent | tBu | 4-$CH_3$ | 0.17 (J) | 165 | 557 (100%) | 56 | XXIX |
| 36 | (R&S) cPent | tBu | 4-Phenyl | 0.35 (J) | 90 (Schaum) | 619 (60%) 57 (100%) | 58 | XXX |
| 37 | (R&S) cPent | tBu | 2-Cl | 0.26 (J) | 156 | 577 (40%) 57 (100%) | 61 | XXXI |
| 38 | (R&S) cPent | tBu | 3-Cl | 0.21 (J) | 135 | 577 (100%) | 40 | II |

| Bsp.-Nr. | $R^1$ | $R^{21}$ | $R^{22}$ | $R_f$ * | F (°C) | Masssen-spektrum | Ausbeute (% d.Th.) | Ausgangs-verbindung |
|---|---|---|---|---|---|---|---|---|
| 39 | (R&S) cPent | tBu | 4-F | 0.17 (J) | 162 | 561 (100%)<br>505 (100%) | 60 | XXXII |
| 40 | (R&S) cPent | tBu | 2-$OCH_3$ | 0.21 (J) | 138 | 573 (40%)<br>57 (100%) | 64 | XXXIII |
| 41 | (R&S) cPent | tBu | 3-$OCH_3$ | 0.19 (J) | --- | 573 (60%)<br>57 (100%) | 72 | XXXIV |
| 42 | (R&S) cPent | tBu | 4-$OCH_3$ | 0.28 (J) | 75<br>(Schaum) | 573 (80%)<br>57 (100%) | 48 | XXXV |

**Tabelle 3:**

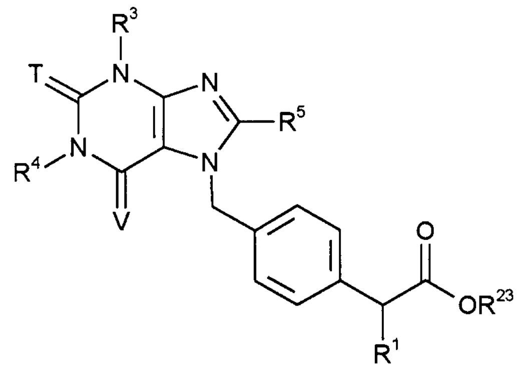

| Bsp.-Nr. | $R^1$ | $R^3$ | $R^4$ | $R^5$ | $R^{23}$ | T | V | $R_f$ * | F (°C) | Massen-spektrum | Ausbeute (% d.Th.) | Ausgangs-verbindung |
|---|---|---|---|---|---|---|---|---|---|---|---|---|
| 43 | (R&S) cPent | $C_2H_5$ | $C_2H_5$ | H | tBu | O | O | 0.28 (J) | Öl | 481 (100%) 57 (90%) | 76 | III |
| 44 | (R&S) cPent | $C_2H_5$ | $C_2H_5$ | $CH_3$ | tBu | O | O | 0.22 (J) | 167 | 495 (50%) 57 (100%) | 66 | XXXVII |
| 45 | (R&S) cPent | $C_2H_5$ | $C_2H_5$ | $C_2H_5$ | tBu | O | O | 0.34 (J) | 121-24 | 509 (100%) 57 (80%) | 69 | XXXVIII |
| 46 | (R&S) cPent | $C_2H_5$ | $C_2H_5$ | cPro | $CH_3$ | O | O | 0.40 (J) | 128 | 479 (80%) 55 (100%) | 49 | XXIX |
| 47 | (R&S) cPent | $C_2H_5$ | $C_2H_5$ | Phenyl | $CH_3$ | O | O | 0.26 (J) | 58 (Schaum) | 515 (100%) | 57 | |
| 48 | (R&S) cPent | $C_2H_5$ | $C_2H_5$ | 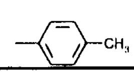 | $CH_3$ | O | O | 0.40 (J) | 58 (Schaum) | 529 (100%) | 46 | XL |
| 49 | (R&S) cPent | $C_2H_5$ | $C_2H_5$ | 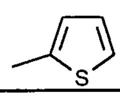 | $CH_3$ | O | O | 0.58 (J) | 166 | 521 (85%) 55 (100%) | 65 | XLI |
| 50 | (R&S) cPent | $CH_3$ | iBu | H | tBu | O | O | 0.64 (J) | --- | 495 (60%) 57 (100%) | 69 | |

| Bsp.-Nr. | $R^1$ | $R^3$ | $R^4$ | $R^5$ | $R^{23}$ | T | V | $R_f$ * | F (°C) | Massen-spektrum | Ausbeute (% d.Th.) | Ausgangs-verbindung |
|---|---|---|---|---|---|---|---|---|---|---|---|---|
| 51 | (R&S) cPent | $CH_3$ | $CH_3$ | $CH_3$ | tBu | S | O | 0.43 (J) | 158-60 | 483 (60%) 427 (100%) 57 (100%) | 68 | XLII |
| 52 | (R&S) cPent | $CH_3$ | $CH_3$ | $C_2H_5$ | tBu | S | O | 0.60 (J) | 178-82 | 497 (80%) 441 (100%) 57 (100%) | 68 | XLIII |
| 53 | (R&S) cPent | $CH_3$ | $CH_3$ | 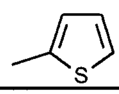 | tBu | S | O | 0.84 (J) | 180-83 | 551 (20%) 57 (100%) | 57 | XLIV |
| 54 | (R&S) cPent | $CH_3$ | $CH_3$ | 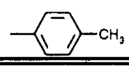 | tBu | S | O | 0.88 (J) | 158-62 | 559 (20%) 57 (100%) | 68 | XLV |

**Beispiel 55**

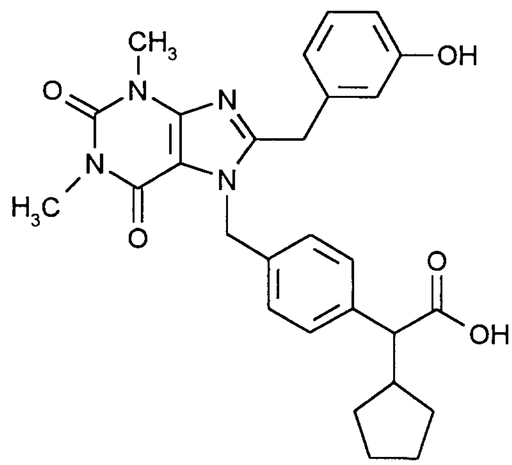

Man löst 5.26 g (9.18 mmol) der Verbindung aus Beispiel 41 in 50 ml $CH_2Cl_2$ und kühlt die Lösung auf -78°C ab. Dazu tropft man 45.9 ml (45.9 mmol; 1 molar in $CH_2Cl_2$) Bortribromid langsam dazu. Das Gemisch wird 2 Stunden bei Raumtemperatur nachgerührt. Man kühlt dieses anschließend mit einem Eisbad wieder auf 0°C ab und versetzt mit 50 ml Methanol. Es wird über Nacht bei Raumtemperatur nachgerührt. Das Lösemittel wird abrotiert, der Rückstand wird in $CH_2Cl_2$ und Wasser aufgenommen und extrahiert. Die organische Phase wird über Natriumsulfat getrocknet, dabei fallen Kristalle aus, die mit Zugabe von wenig Methanol wieder in Lösung gehen. Das Natriumsulfat wird abgesaugt, die Mutterlauge wird einrotiert. Der Rückstand (Kristalle) wird in $CH_2Cl_2$ verrührt und abgesaugt.

| | |
|---|---|
| Ausbeute | (36 %) |
| $R_f$ = | 0,27 (Dichlormethan:Methanol, 20:1) |
| Fp= | 160°C |

In Analogie zur Vorschrift des Beispiels 55 werden die in Tabelle 4 aufgeführten Verbindungen hergestellt:

**Tabelle 4:**

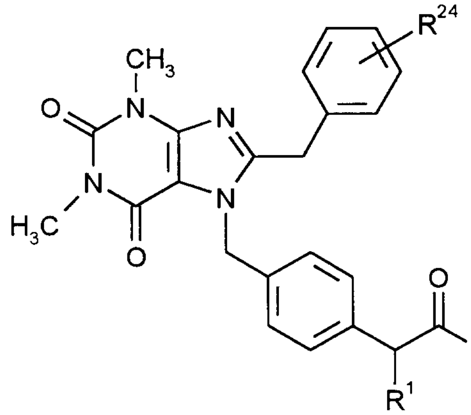

| Bsp.-Nr. | $R^1$ | $R^{24}$ | $R_f$ | Mp (°C) | Massen-spektrum | Ausbeute (% d.Th.) | Ausgangs-verbindung |
|---|---|---|---|---|---|---|---|
| 56 | (R&S) cPent | 2-OH | 0.31 (K) | (Schaum) | 503 (100%) | 29 | 40 |
| 57 | (R&S) cPent | 4-OH | 0.16 (K) | 100 (Schaum) | 503 (100%) | 73 | 42 |

**Beispiel 58**

2-(R&S)Cyclopentyl-[4-(1,3-dimethyl-2,6-dioxo-8-(4-methyl)phenyl-1,2,3,6-tetrahydro-purin-7-ylmethyl)phenyl]essigsäure Hydrochlorid

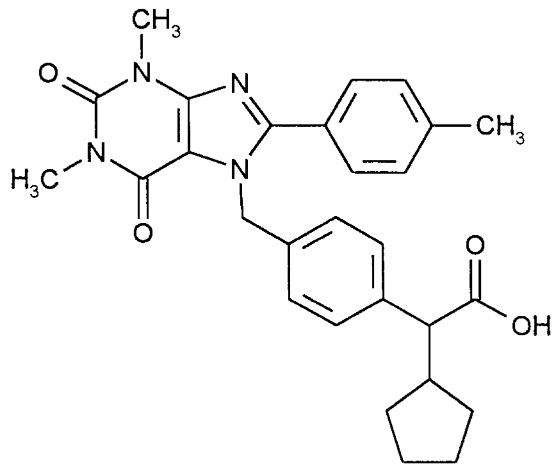

8.3 g (15.3 mmol) der Verbindung aus Beispiel 1 wurden in Dioxan (60 ml) gelöst, mit konzentrierter Salzsäure versetzt und 4 h beim Rückfluß gekocht. Es wurde abgekühlt und mit kaltem Wasser versetzt. Es wurde 20 Minuten nachgerührt, und die anfallende Kristalle wurden abgesaugt.
Ausbeute 7.21 g (97%);
FP >240 °C;

$R_f$ = 0.23 (Dichlormethan : Methanol, 20 : 1);
Masse (berechnet) für $C_{28}H_{30}N_4O_4$ = 486.58, Massenspektrum (FAB, rel. Intensität) 487 (100%);
$^1H$ NMR (300 MHz, $CDCl_3$) δ 7.45 (d, J = 8.19 Hz, 2 H), 7.28-7.23 (m, 4 H), 6.98 (d, J = 8.18 Hz, 2 H), 5.60 (s, 2 H), 3.64 (s, 3 H), 3.40 (s, 3 H), 3.23 (d, J = 11.08 Hz, 1 H), 2.41 (m, 1 H), 2.40 (s, 3 H), 1.92 (m, 1 H), 1.70-1.20 (m, 6 H), 0.97 (m, 1 H).

Die in den Tabellen 5, 6 und 7 aufgeführten Verbindungen werden in Analogie zur Vorschrift des Beispiels 58 hergestellt:

**Tabelle 5:**

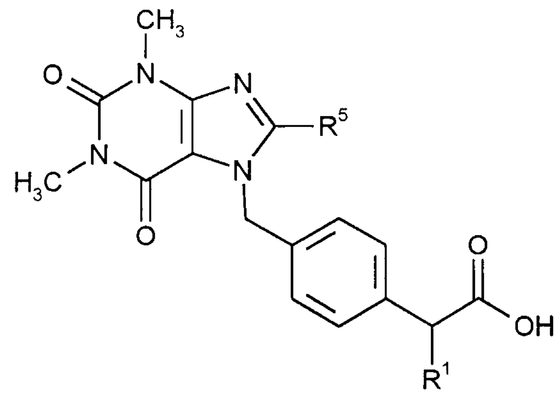

| Bsp.-Nr. | $R^1$ | $R^5$ | $R_f$ * | F (°C) | Massen-spektrum | Ausbeute (% d.Th.) | Ausgangs-verbindung |
|---|---|---|---|---|---|---|---|
| 59 | (R&S) cPent | H | 0.27 (K) | 94 (Schaum) | *397 (40%) | 76 | 2 |
| 60 | (R&S)cPent | Br | 0.35 (K) | 192 | | 82 | 3 |
| 61 | (R&S)cPent | $CH_3$ | 0.22 (K) | >230 | 411 (100%) | 100 | 4 |
| 62 | (R&S)cPent | $C_2H_5$ | 0.20 (K) | 197 | 425 (100%) | 64 | 5 |
| 63 | (R&S)cPent | cPro | 0.29 (K) | 94 | 437 (100%) | 100 | 6 |
| 64 | (R&S)cPent | cPent | 0.47 (K) | 96 (Schaum) | 465 (100%) | 83 | 7 |
| 65 | (R&S)cPent | $-CH_2cHex$ | 0.38 (K) | 217 | *493 (100%) | 74 | 8 |
| 66 | (R&S)cPent | 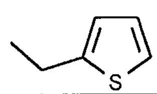 | 0.31 (K) | 190 | 493 (100%) | 41 | 9 |

| Bsp.-Nr. | $R^1$ | $R^5$ | $R_f$ * | F (°C) | Massen-spektrum | Ausbeute (% d.Th.) | Ausgangs-verbindung |
|---|---|---|---|---|---|---|---|
| 67 | (R&S)cPent | 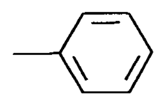 | 0.39 (J) | | | 100 | 10 |
| 68 | (R&S)cPent | 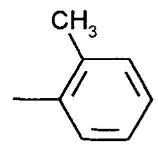 | 0.28 (K) | 185 | 487 (100%) | 100 | 11 |
| 69 | (R&S)cPent | 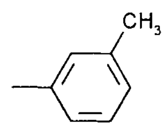 | 0.24 (K) | 155 | 487(100%) | 84 | 12 |
| 70 | (R&S)cPent | 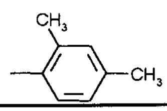 | 0.35 (K) | 165 | 501 (100%) | 96 | 13 |
| 71 | (R&S)cPent | 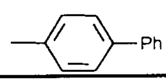 | 0.36 (K) | 218 | 549 (100%) | 56 | 14 |
| 72 | (R&S)cPent | 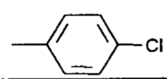 | 0.33 (K) | 130 (Schaum) | 507 (100%) | 67 | 15 |
| 73 | (R&S)cPent | 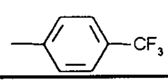 | 0.31 (K) | 132 | 541 (80%) 149 (100%) | 85 | 16 |
| 74 | (R&S)cPent | 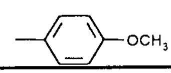 | 0.35 (K) | 122 (Schaum) | 503 (100%) | 88 | 17 |
| 75 | (R&S)cPent | 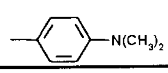 | 0.29 (K) | >245 | *516 (70%) 307 (100%) | 88 | 18 |
| 76 | (R&S)cPent | 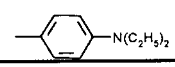 | 0.46 (K) | (Schaum) | 544 (100%) | 100 | 19 |

| Bsp.-Nr. | $R^1$ | $R^5$ | $R_f$ * | F (°C) | Massen-spektrum | Ausbeute (% d.Th.) | Ausgangs-verbindung |
|---|---|---|---|---|---|---|---|
| 77 | (R&S)cPent | 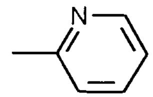 | 0.23 (K) | >240 | 474 (100%) | 83 | 20 |
| 78 | (R&S)cPent | 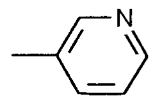 | 0.52 (L) | --- | 474 (100%) | 27 | 21 |
| 79 | (R&S)cPent | 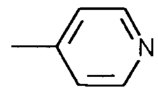 | 0.25 (L) | >240 | 474 (100%) | 72 | 22 |
| 80 | (R&S)cPent | 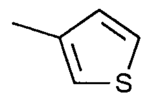 | 0.23 (K) | 217 | 479 (100%) | 66 | 23 |
| 81 | (R&S)cPent | 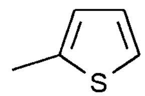 | 0.34 (K) | 215-16 | 479 (100%) | 45 | 24 |
| 82 | (R&S)cPent | 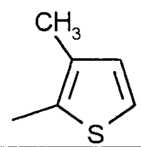 | 0.32 (K) | >235 | 493 (100%) | 100 | 25 |
| 83 | (R&S)cPent | 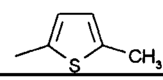 | 0.23 (K) | 112 (Schaum) | 493 (100%) | 81 | 26 |
| 84 | (R&S)cPent | 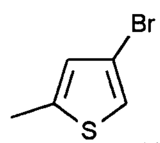 | 0.35 (K) | >235 | 557 (100%) | 100 | 27 |
| 85 | (R&S)cPent | 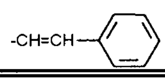 | 0.35 (K) | >240 | 476 (50%) | | 28 |

**Tabelle 6:**

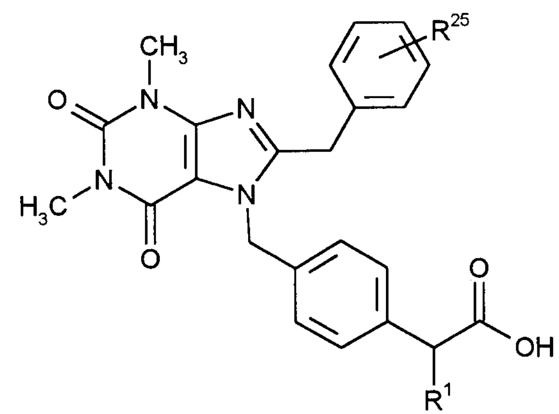

| Bsp.-Nr. | $R^1$ | $R^{25}$ | $R_f$ * | F (°C) | Masssen-spektrum | Ausbeute (% d.Th.) | Ausgangs-verbindung |
|---|---|---|---|---|---|---|---|
| 86 | (R&S) cPent | H | 0.44 (K) | 97 | 487 (100%) | 90 | 29 |
| 87 | (R&S) cHex | H | 0.29 (K) | 117 (Schaum) | 501 (100%) | 55 | 30 |
| 88 | (R&S) cHept | H | 0.41 (K) | 214 | *514 (100%) | 60 | 31 |
| 89 | (R&S)Ph | H | 0.22 (K) | 130 | 495 (100%) | 42 | 32 |
| 90 | (R&S) cPent | 2-$CH_3$ | 0.42 (K) | 192 | 501 (100%) | 94 | 33 |
| 91 | (R&S) cPent | 3-$CH_3$ | 0.24 (K) | 196 | 501 (100%) | 91 | 34 |
| 92 | (R&S) cPent | 4-$CH_3$ | 0.27 (K) | 222 | 501 (100%) | 97 | 35 |
| 93 | (R&S) cPent | 4-Ph | 0.31 (K) | 215 | 563 (100%) | 100 | 36 |
| 94 | (R&S) cPent | 2-C1 | 0.34 (K) | 155 | 521 (100%) | 100 | 37 |
| 95 | (R&S) cPent | 3-Cl | 0.25 (K) | 194 | 521 (100%) | 68 | 38 |
| 96 | (R&S) cPent | 4-F | 0.25 (K) | 213 | 505 (100%) | 94 | 39 |

| Bsp.-Nr. | $R^1$ | $R^{25}$ | $R_f$ * | F (°C) | Masssen-spektrum | Ausbeute (% d.Th.) | Ausgangs-verbindung |
|---|---|---|---|---|---|---|---|
| 97 | (R&S) cPent | 2-$OCH_3$ | 0.28 (K) | 63 | 517 (100%) | 100 | 40 |
| 98 | (R&S) cPent | 3-$OCH_3$ | 0.22 (K) | 188 | 517 (100%) | 64 | 41 |
| 99 | (R&S) cPent | 4-$OCH_3$ | 0.26 (K) | 210 (Schaum) | 517 (100%) | 92 | 42 |

**Tabelle 7:**

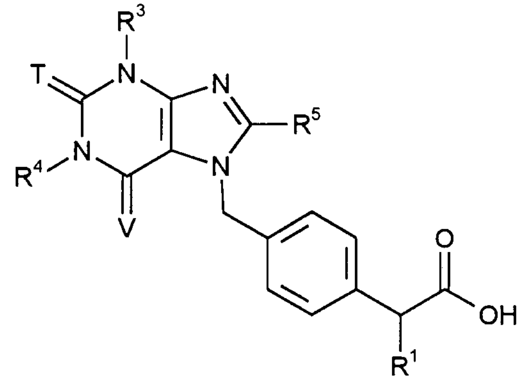

| Bsp.-Nr. | $R^1$ | $R^3$ | $R^4$ | $R^5$ | T | V | $R_f$ * | F (°C) | Massen-spektrum | Ausbeute (% d.Th.) | Ausgangs-verbindung |
|---|---|---|---|---|---|---|---|---|---|---|---|
| 100 | (R&S) cPent | $C_2H_5$ | $C_2H_5$ | H | O | O | 0.27 (K) | 110 | 425 (100%) | 100 | 43 |
| 101 | (R&S) cPent | $C_2H_5$ | $C_2H_5$ | $CH_3$ | O | O | 0.24 (K) | 82-85 | 439 (100%) | 100 | 44 |
| 102 | (R&S) cPent | $C_2H_5$ | $C_2H_5$ | $C_2H_5$ | O | O | 0.20 (K) | 181 | 453 (100%) | 96 | 45 |
| 103 | (R&S) cPent | $C_2H_5$ | $C_2H_5$ | cPro | O | O | 0.30 (K) | 98 (Schaum) | 465 (100%) | 78 | 46 |
| 104 | (R&S) cPent | $C_2H_5$ | $C_2H_5$ | Ph | O | O | 0.38 (K) | 100 (Schaum) | 501 (100%) | 80 | 47 |
| 105 | (R&S) cPent | $C_2H_5$ | $C_2H_5$ | 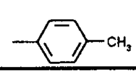 | O | O | 0.16 (K) | 92 (Schaum) | 515 (100%) | 75 | 48 |
| 106 | (R&S) cPent | $C_2H_5$ | $C_2H_5$ | 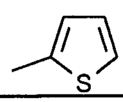 | O | O | 0.33 (K) | 232 | 507 (80%) 464 (100%) | 85 | 49 |

| Bsp.-Nr. | $R^1$ | $R^3$ | $R^4$ | $R^5$ | T | V | $R_f$ * | F (°C) | Massen-spektrum | Ausbeute (% d.Th.) | Ausgangs-verbindung |
|---|---|---|---|---|---|---|---|---|---|---|---|
| 107 | (R&S) cPent | $CH_3$ | iBu | H | O | O | 0.33 (K) | 76 (Schaum) | 439 (100%) | 98 | 50 |
| 108 | (R&S) cPent | $CH_3$ | $CH_3$ | $CH_3$ | S | O | 0.12 (J) | 222 | 427 (100%) | 100 | 51 |
| 109 | (R&S) cPent | $CH_3$ | $CH_3$ | $C_2H_5$ | S | O | 0.14 (J) | 172 | 441 (100%) | 100 | 52 |
| 110 | (R&S) cPent | $CH_3$ | $CH_3$ | 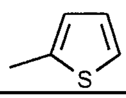 | S | O | 0.15 (J) | >235 | 495 (100%) | 100 | 53 |
| 111 | (R&S) cPent | $CH_3$ | $CH_3$ | 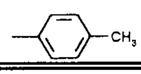 | S | O | 0.26 (J) | >235 | 503 (40%) 55 (100%) | 86 | 54 |

**Beispiel 112**

N-[2-(R)-Phenyl-1-hydroxyethan]-2-(R&S)-cyclopentyl-[4-(1,3-dimethyl)-2,6-dioxo-8-(4-methyl)phenyl-1,2,3,6-tetrahydro-purin-7-ylmethyl)phenyl]essigsäureamid

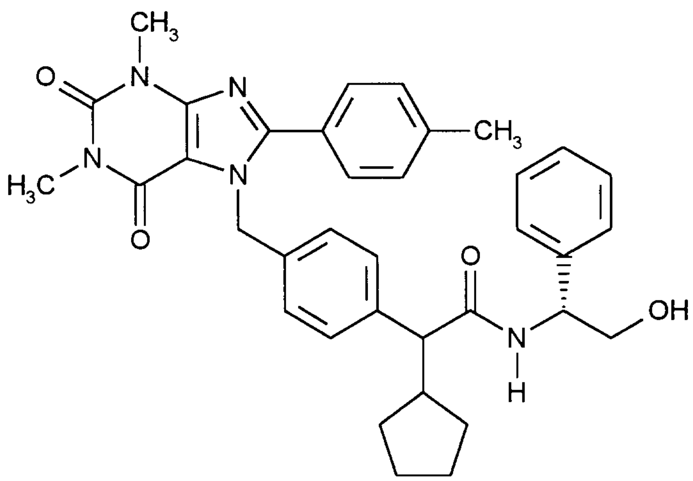

6.1 g (12.5 mmol) der Verbindung aus Beispiel 58, (R)-Phenylglycinol (1.71 g, 12.5 mmol), 1-Hydroxy-1-benzotriazol (1.86 g, 13.8 mmol), N'-(3-Dimethylaminopropyl)-N-ethylcarbodiimid Hydrochlorid (2.76 g, 14.4 mmol) und Triethylamin (2.53 g, 25 mmol) werden nacheinander in $CH_2Cl_2$ (60 ml) gelöst und über Nacht bei Raumtemperatur gerührt. Es werden nochmal 15 ml $CH_2Cl_2$ zugegeben und mit wäßriger $NH_4Cl$- und $NaHCO_3$-Lösung sowie mit Wasser gewaschen. Die organische Phase wird über Natriumsulfat getrocknet und eingedampft. Der Rückstand wird säulenchromatographisch gereinigt.

Ausbeute 6.59 g (87%);

Fp 87 °C (Schaum);

$R_f$ = 0.32 (Dichlormethan : Methanol, 20 : 1);

Masse (berechnet) für $C_{36}H_{39}N_5O_4$ = 605.75, Massenspektrum (FAB, rel. Intensität) 606 (100%), 105 (95%);

Die in Tabellen 8, 9 und 10 aufgeführten Verbindungen werden in Analogie zur Vorschrift des Beispiels 112 hergestellt. Entweder fallen die Verbindungen direkt als reine Diastereomere an, oder sie werden ausgehend vom Racemat nach üblichen Methoden säulenchromatographisch getrennt.

**Tabelle 8:**

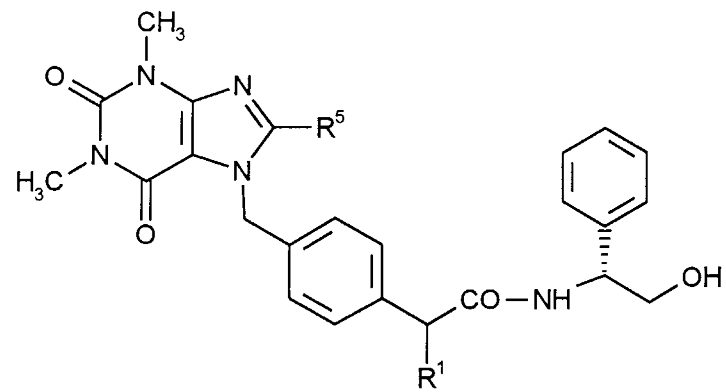

| Bsp.-Nr. | $R^1$ | $R^5$ | $R_f$ * | F (°C) | Massen-spektrum | Ausbeute (% d.Th.) | Ausgangs-verbindung |
|---|---|---|---|---|---|---|---|
| 113 | (R&S) cPent | H | 0.27 (K) | 96 (Schaum) | 516 (100%) | 70 | 59 |
| 114 | (dia A) cPent | H | 0.27 (K) | (Schaum) | | | 113 |
| 115 | (dia B) cPent | H | 0.25 (K) | 162-163 | | | 113 |
| 116 | (R&S) cPent | Br | 0.33 (K) | 109 (Schaum) | 596 (35%) 171 (100%) | 88 | 60 |
| 117 | (dia A) cPent | Br | | 183-185 | 596 (35%) 572 (100%) | | 116 |
| 118 | (dia B) cPent | Br | | 118-23 | 596 (35%) 550 (100%) | | 116 |
| 119 | (R&S) cPent | $CH_3$ | 0.35 (K) | 126-28 | 530 (60%) 105 (100%) | 85 | 61 |
| 120 | (R&S) cPent | $C_2H_5$ | 0.27 (K) | 105 (Schaum) | 544 (100%) | 72 | 62 |

| Bsp.-Nr. | $R^1$ | $R^5$ | $R_f$ * | F (°C) | Massen-spektrum | Ausbeute (% d.Th.) | Ausgangs-verbindung |
|---|---|---|---|---|---|---|---|
| 121 | (R&S) cPent | cPro | 0.36 (K) | 110 (Schaum) | 556 (80%) 105 (100%) | 73 | 63 |
| 122 | (R&S) cPent | cPent | 0.33 (K) | 194 | 584 (100%) | 65 | 64 |
| 123 | (R&S) cPent | $-CH_2cHex$ | 0.31 (K) | 117 (Schaum) | 612 (100%) | 69 | 65 |
| 124 | (dia A) cPent | $-CH_2cHex$ | 0.46 (K) | 173 | C, HN Pent | | 123 |
| 125 | (dia B) cPent | $-CH_2cHex$ | 0.46 (K) | 90 (Schaum) | 612 (100 %) | | 123 |
| 126 | (R&S)cPent | 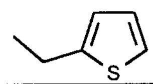 | 0.35 (K) | 112 (Schaum) | 612 (100%) | 81 | 66 |
| 127 | (dia A)cPent | 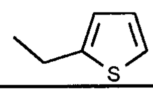 | 0.16 (K) | 183 | 612 (50 %) 227 (100 %) | | 126 |
| 128 | (dia B)cPent | 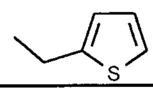 | 0.16 (K) | 82 (Schaum) | 612 (40 %) 105 (100 %) | | 126 |
| 129 | (R&S)cPent | 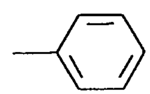 | 0.50 (L) | | | 98 | 67 |
| 130 | (dia A) cPent | 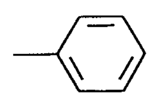 | 0.50 (L) | | 592 (100%) | | 129 |
| 131 | (dia B) cPent | 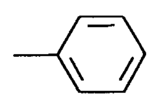 | 0.50 (L) | | 592 (100%) | | 129 |
| 132 | (R&S)cPent | 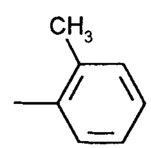 | 0.31 (K) | 128 (Schaum) | 606 (100%) | 79 | 68 |

| Bsp.-Nr. | $R^1$ | $R^5$ | $R_f$ * | F (°C) | Massen-spektrum | Ausbeute (% d.Th.) | Ausgangs-verbindung |
|---|---|---|---|---|---|---|---|
| 133 | (R&S)cPent | 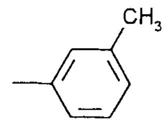 | 0.25 (K) | 115 (Schaum) | 606 (100%) | 81 | 69 |
| 134 | (dia A) cPent | 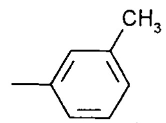 | 0.23 (K) | 189-91 | 606 (50 %)<br>171 (100%) | | 133 |
| 135 | (dia B) cPent | 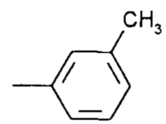 | 0.25 (K) | 154 | 606 (100 %) | | 133 |
| 136 | (dia A) cPent | 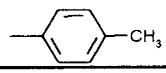 | 0.46 (K) | 104 (Schaum) | | | 112 |
| 137 | (dia B) cPent | 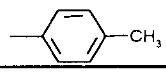 | 0.46 (K) | 198 (Schaum) | | | 112 |
| 138 | (R&S)cPent | 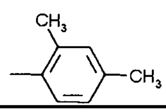 | 0.25 (K) | 132 (Schaum) | 620 (100%) | 85 | 70 |
| 139 | (R&S)cPent | 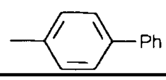 | 0.28 (K) | 105 (Schaum) | 668 (70%)<br>154 (100%) | 86 | 71 |
| 140 | (R&S)cPent | 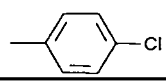 | 0.34 (K) | 132 (Schaum) | 626 (100%) | 85 | 72 |
| 141 | (dia A) cPent | 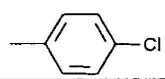 | 0.45 (K) | 80 (Schaum) | | | 140 |
| 142 | (dia B) cPent | 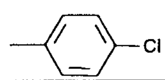 | 0.45 (K) | 198 | | | 140 |

| Bsp.-Nr. | $R^1$ | $R^5$ | $R_f$ * | F (°C) | Massen-spektrum | Ausbeute (% d.Th.) | Ausgangs-verbindung |
|---|---|---|---|---|---|---|---|
| 143 | (R&S) cPent | 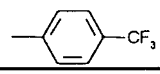 | 0.34 (K) | 130 (Schaum) | 660 (100%) | 69 | 73 |
| 144 | (dia A) cPent | 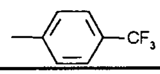 | 0.47 (K) | 245 | 660 (50 %) 171 (100 %) | | 143 |
| 145 | (dia B) cPent | 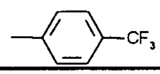 | 0.45 (K) | 212 | C,H,N Anal. | | 143 |
| 146 | (R&S) cPent | 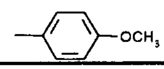 | 0.20 (K) | 126 (Schaum) | 622 (100%) | 84 | 74 |
| 147 | (dia A) cPent | 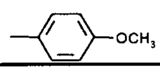 | 0.43 (K) | Öl | 622 (351) 105 (100 %) | | 146 |
| 148 | (dia B) cPent | 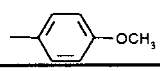 | 0.45 (K) | 201 | C,H,N Anal. | | 146 |
| 149 | (R&S) cPent | 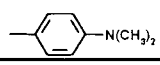 | 0.25 (K) | 140 (Schaum) | 635 (100%) | 13 | 75 |
| 150 | (R&S) cPent | 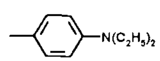 | 0.24 (K) | 128 (Schaum) | 663 (100%) | 72 | 76 |
| 151 | (R&S) cPent | 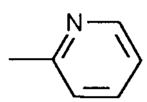 | 0.28 (K) | >240 | 593 (100%) | 64 | 77 |
| 152 | (R&S) cPent | 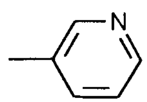 | 0.14 (K) | 123 (Schaum) | 593 (100%) | 81 | 78 |
| 153 | (R&S) cPent | 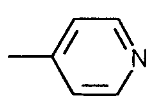 | | | | | 79 |
| 154 | (R&S) cPent | 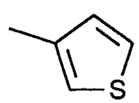 | 0.21 (K) | 115 (Schaum) | 598 (60%) 105 (100%) | 82 | 80 |

| Bsp.-Nr. | $R^1$ | $R^5$ | $R_f$ * | F (°C) | Massen-spektrum | Ausbeute (% d.Th.) | Ausgangs-verbindung |
|---|---|---|---|---|---|---|---|
| 155 | (dia A) cPent | 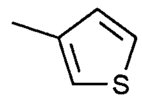 | 0.43 (K) | 174-76 | 598 (100%) | | 154 |
| 156 | (dia B) cPent | 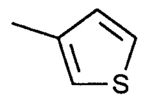 | 0.49 (K) | 197-99 | 598 (100%) | | 154 |
| 157 | (R&S) cPent | 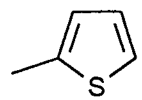 | 0.30 (K) | (Schaum) | 598 (30%)<br>307 (100%) | 49 | 81 |
| 158 | (dia A) cPent | 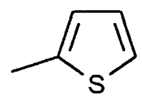 | 0.34 (K) | 136 | | | 157 |
| 159 | (dia B) cPent | 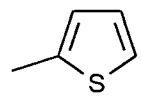 | 0.31 (K) | 128 | | | 157 |
| 160 | (R&S) cPent | 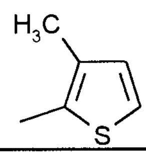 | 0.46 (K) | 155 | 612 (100%) | 60 | 82 |
| 161 | (dia A) cPent | 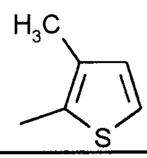 | 0.41 (K) | 123 | 612 (100 %) | | 160 |
| 162 | (dia B) cPent | 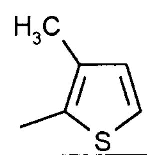 | 0.37 (K) | 187 | C,H,N Anal. | | 160 |

| Bsp.-Nr. | $R^1$ | $R^5$ | $R_f$ * | F (°C) | Massen-spektrum | Ausbeute (% d.Th.) | Ausgangs-verbindung |
|---|---|---|---|---|---|---|---|
| 163 | (R&S) cPent | 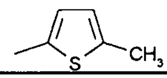 | 0.39 (J) | 97 (Schaum) | 612 (60%) 57 (100%) | 85 | 83 |
| 164 | (R&S) cPent | 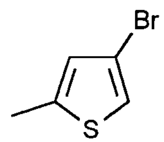 | 0.48 (K) | >235 | 676 (20%) 55 (100%) | 61 | 84 |
| 165 | (dia A) cPent | 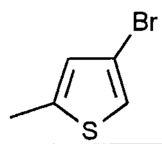 | 0.37 (K) | 175 | C,H,N Anal. | | 164 |
| 166 | (dia B) cPent | 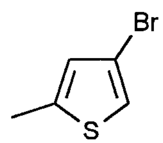 | 0.37 (K) | 169 | 676 (100 %) | | 164 |
| 167 | (R&S)cPent | 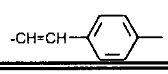 | 0.49 (K) | 160-69 | 618 (100%) | 31 | 85 |

**Tabelle 9:**

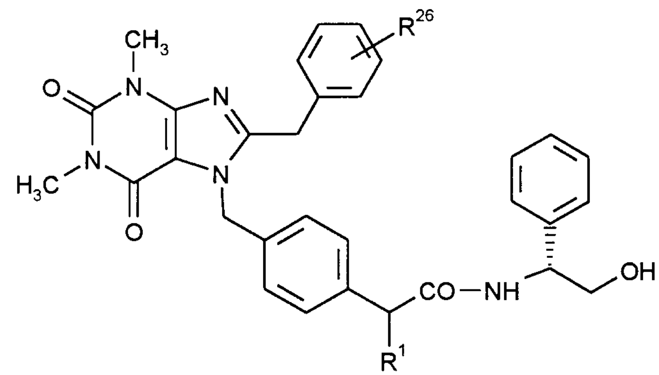

| Bsp.-Nr. | $R^1$ | $R^{26}$ | $R_f$ * | F (°C) | Massen-spektrum | Ausbeute (% d.Th.) | Ausgangs-verbindung |
|---|---|---|---|---|---|---|---|
| 168 | (R&S) cPent | H | 0.22 (K) | (Schaum) | 606 (100%) | 98 | 86 |
| 169 | (dia A) cPent | H | | 204-7 | | | 168 |
| 170 | (dia B) cPent | H | | (Schaum) | | | 168 |
| 171 | (R&S) cHex | H | 0.40 (K) | | 620 (100%) | 86 | 87 |
| 172 | (dia A) cHex | H | 0.33 (K) | 226 | C,H,N Anal. | | 171 |
| 173 | (dia B) cHex | H | 0.34 (K) | 116 | 620 (45 %)<br>185 (100 %) | | 171 |
| 174 | (R&S) cHept | H | 0.36 (K) | 108<br>(Schaum) | | 92 | 88 |
| 175 | (dia A) cHept | H | | 202 | | | 174 |
| 176 | (dia B) cHept | H | | 100<br>(Schaum) | | | 174 |
| 177 | (R&S) Ph | H | 0.15 (K) | 106-10 | 614 (100 %) | 50 % | 89 |

| Bsp.-Nr. | $R^1$ | $R^{26}$ | $R_f$ * | F (°C) | Massen-spektrum | Ausbeute (% d.Th.) | Ausgangs-verbindung |
|---|---|---|---|---|---|---|---|
| 178 | (R&S) cPent | 2-$CH_3$ | 0.38 (K) | 137 | 620 (100%) | 54 | 90 |
| 179 | (R&S) cPent | 3-$CH_3$ | 0.28 (K) | 121 (Schaum) | 620 (100%) | 36 | 91 |
| 180 | (dia A) cPent | 3-$CH_3$ | 0.16 (K) | 200 | C,H,N Anal. | | 179 |
| 181 | (dia B) cPent | 3-$CH_3$ | 0.17 (K) | 166 | | | 179 |
| 182 | (R&S) cPent | 4-$CH_3$ | 0.33 (K) | 113 (Schaum) | 620 (100%) | 92 | 92 |
| 183 | (dia A) cPent | 4-$CH_3$ | 0.37 (K) | 223 | C,H,N Anal. | | 182 |
| 184 | (dia B) cPent | 4-$CH_3$ | 0.40 (K) | 208 | C,H,N Anal. | | 182 |
| 185 | (R&S) cPent | 4-Phenyl | 0.49 (K) | 180 | 682 (100%) | 47 | 93 |
| 186 | (dia A) cPent | 4-Phenyl | 0.32 (K) | >230 | C,H,N Anal. | | 185 |
| 187 | (dia B) cPent | 4-Phenyl | 0.31 (K) | 200 | C,H,N Anal. | | 185 |
| 188 | (R&S) cPent | 2-Cl | 0.39 (K) | 113 (Schaum) | 640 (100%) | 77 | 94 |
| 189 | (dia A) cPent | 2-Cl | 0.38 (K) | 208 | C,H,N Anal. | | 188 |
| 190 | (dia B) cPent | 2-Cl | 0.34 (K) | 142 | C,H,N Anal. | | 188 |
| 191 | (R&S) cPent | 3-Cl | 0.41 (K) | 142 | 640 (100%) | 86 | 95 |
| 192 | (dia A) cPent | 3-Cl | 0.35 (K) | 200 | C,H,N Anal. | | 191 |
| 193 | (dia B) cPent | 3-Cl | 0.35 (K) | 183 | | | 191 |
| 194 | (R&S) cPent | 4-F | 0.42 (K) | 151 (Schaum) | 624 (100%) | 81 | 96 |
| 195 | (dia A) cPent | 4-F | 0.38 (K) | 212 | C,H,N Anal. | | 194 |
| 196 | (dia B) cPent | 4-F | 0.37 (K) | 189 | C,H,N Anal. | | 194 |
| 197 | (R&S) cPent | 2-$OCH_3$ | 0.37 (K) | 120 (Schaum) | 636 (100%) | 61 | 97 |

| Bsp.-Nr. | $R^1$ | $R^{26}$ | $R_f$ * | F (°C) | Massen-spektrum | Ausbeute (% d.Th.) | Ausgangs-verbindung |
|---|---|---|---|---|---|---|---|
| 198 | (R&S) cPent | 3-$OCH_3$ | 0.48 (K) | 105 (Schaum) | 636 (100%) | 79 | 98 |
| 199 | (dia A) cPent | 3-$OCH_3$ | 0.19 (K) | 188 | | | 198 |
| 200 | (dia B) cPent | 3-$OCH_3$ | 0.15 (K) | 178 | | | 198 |
| 201 | (R&S) cPent | 4-$OCH_3$ | 0.35 (K) | 110 (Schaum) | 636 (100%) | 70 | 99 |
| 202 | (dia A) cPent | 4-$OCH_3$ | 0.29 (K) | | | | 201 |
| 203 | (dia B) cPent | 4-$OCH_3$ | 0.28 (K) | | | | 201 |
| 204 | (R&S) cPent | 2-OH | 0.23 (K) | 130 | 622 (100%) | 67 | 56 |
| 205 | (dia A) cPent | 2-OH | 0.26 (K) | Öl | 622 (40 %) 105 (100 %) | | 204 |
| 206 | (dia B) cPent | 2-OH | 0.26 (K) | Öl | 622 (50 %) 105 (100 %) | | 204 |
| 207 | (R&S) cPent | 3-OH | 0.14 (K) | 128 (Schaum) | 622 (100%) | 70 | 55 |
| 208 | (dia A) cPent | 3-OH | 0.38 (K) | 143 | 622 (100%) | | 207 |
| 209 | (dia B) cPent | 3-OH | 0.38 (K) | 145 | 622 (100%) | | 207 |
| 210 | (R&S) cPent | 4-OH | | | | | 57 |

Tabelle 10:

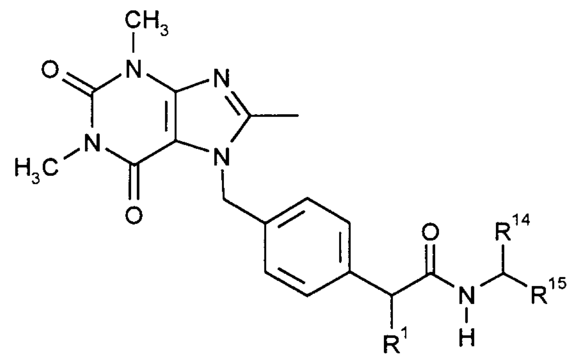

| Bsp.-Nr. | $R^1$ | $R^3$ | $R^4$ | $R^5$ | $R^{14}$ | $R^{15}$ | T | V | $R_f$* | F (°C) | Massen-spektrum | Ausbeute (% d.Th.) | Ausgangs-verbindung |
|---|---|---|---|---|---|---|---|---|---|---|---|---|---|
| 211 | (R&S) cPent | $C_2H_5$ | $C_2H_5$ | H | (R) Ph | $CH_2OH$ | O | O | 0.40 (J) | | 544 (100%) | 67 | 100 |
| 212 | (dia A) cPent | $C_2H_5$ | $C_2H_5$ | H | (R) Ph | $CH_2OH$ | O | O | 0.40 (J) | 110 | | | 211 |
| 213 | (dia B) cPent | $C_2H_5$ | $C_2H_5$ | H | (R) Ph | $CH_2OH$ | O | O | 0.35 (J) | 121 | | | 211 |
| 214 | (R&S) cPent | $C_2H_5$ | $C_2H_5$ | $CH_3$ | (R) Ph | $CH_2OH$ | O | O | 0.48 (K) | 112-14 | 558 (100%) | 83 | 101 |
| 215 | (R&S) cPent | $C_2H_5$ | $C_2H_5$ | $C_2H_5$ | (R) Ph | $CH_2OH$ | O | O | 0.20 (J) | 104 | 572 (80%) | 84 | 102 |
| 216 | (R&S) cPent | $C_2H_5$ | $C_2H_5$ | cPro | (R) Ph | $CH_2OH$ | O | O | 0.36 (K) | 104 (Schaum) | 584 (100%) | 87 | 103 |
| 217 | (R&S) cPent | $C_2H_5$ | $C_2H_5$ | Ph | (R) Ph | $CH_2OH$ | O | O | 0.31 (K) | 115 (Schaum) | 620 (100%) | 87 | 104 |
| 218 | (R&S) cPent | $C_2H_5$ | $C_2H_5$ | 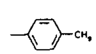 | (R) Ph | $CH_2OH$ | O | O | 0.44 (K) | 168 | 634 (90%) 105 (100%) | 90 | 105 |

| Bsp.-Nr. | $R^1$ | $R^3$ | $R^4$ | $R^5$ | $R^{14}$ | $R^{15}$ | T | V | $R_f$* | F (°C) | Massen-spektrum | Ausbeute (% d.Th.) | Ausgangs-verbindung |
|---|---|---|---|---|---|---|---|---|---|---|---|---|---|
| 219 | (R&S) cPent | $C_2H_5$ | $C_2H_5$ | 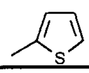 | (R) Ph | $CH_2OH$ | O | O | 0.38 (K) | 115-17 (Schaum) | 626 (70%) 105 (100%) | 90 | 106 |
| 220 | (R&S) cPent | $CH_3$ | iBu | H | (R) Ph | $CH_2OH$ | O | O | 0.39 (K) | 101 (Schaum) | 558 (100%) | 79 | 107 |
| 221 | (dia A) cPent | $CH_3$ | iBu | H | (R) Ph | $CH_2OH$ | O | O | 0.24 (K) | 134 | 558 (100%) | | 220 |
| 222 | (dia B) cPent | $CH_3$ | iBu | H | (R) Ph | $CH_2OH$ | O | O | 0.20 (K) | 186 | C,H,N Anal. | | 220 |
| 223 | (R&S) cPent | $CH_3$ | $CH_3$ | $CH_3$ | (R) Ph | $CH_2OH$ | S | O | 0.45 (J) | 120 (Schaum) | 546 (90%) | 68 | 108 |
| 224 | (R&S) cPent | $CH_3$ | $CH_3$ | $C_2H_5$ | (R) Ph | $CH_2OH$ | S | O | 0.45 (K) | 113 (Schaum) | 560 (100%) | 58 | 109 |
| 225 | (R&S) cPent | $CH_3$ | $CH_3$ | 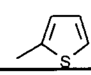 | (R) Ph | $CH_2OH$ | S | O | 0.64 (J) | 130 (Schaum) | 614 (90%) 154 (100%) | 46 | 110 |
| 226 | (R&S) cPent | $CH_3$ | $CH_3$ | 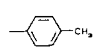 | (R) Ph | $CH_2OH$ | S | O | 0.41 (J) | 104 (Schaum | 622 (100%) | 79 | 111 |

**Beispiel 227**

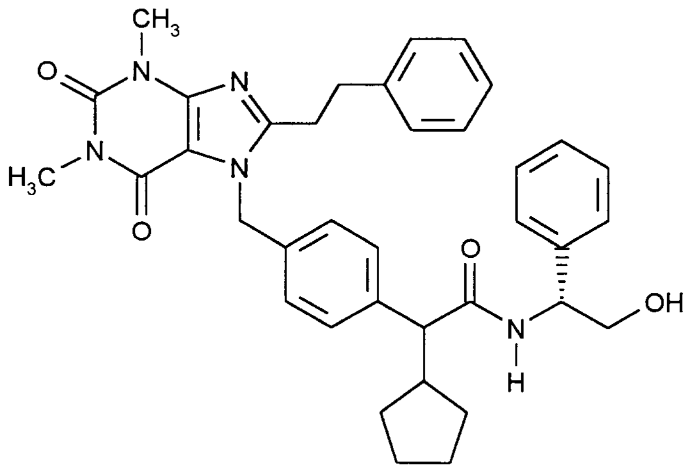

Man löst 0.40 g (0.648 mmol) der Verbindung aus Beispiel 167 in 10 ml Methanol und 10 ml Essigsäure. Dazu gibt man eine Spatelspitze Palladium-Kohle (10%ig) und hydriert 4 Stunden unter Normaldruck. Das Gemisch wird über Celite abgesaugt und einrotiert. Der Rückstand wird in $CH_2Cl_2$ und Wasser aufgenommen, mit Natriumhydrogencarbonat auf pH 8 gestellt und extrahiert. Die organische Phase wird über Natriumsulfat getrocknet und einrotiert. Der Rückstand wird säulenchromatographisch gereinigt.
Ausbeute: 0.180 g (44.9%)
$R_f$ = 0.16 (Dichlormethan : Methanol 20:1)
Masse (berechnet) für $C_{37}H_{41}N_5O_4$ = 619.77

**Patentansprüche**

1. Substituierte Xanthine der allgemeinen Formel

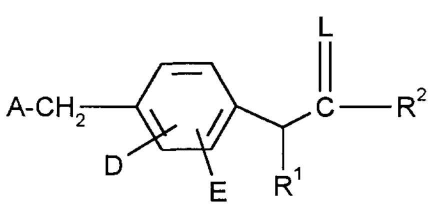

(I)

in welcher

A für einen Rest der Formel

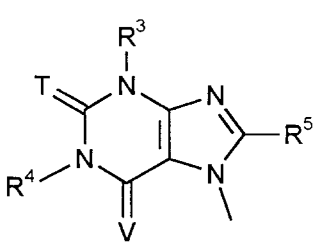

oder

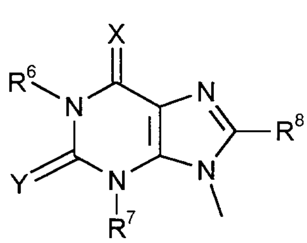

steht,
worin

| | |
|---|---|
| $R^3$, $R^4$, $R^6$ und $R^7$ | gleich oder verschieden sind und Wasserstoff, Cycloalkyl mit 3 bis 7 Kohlenstoffatomen oder Aryl mit 6 bis 10 Kohlenstoffatomen bedeuten, oder geradkettiges oder verzweigtes Alkyl oder Alkenyl mit jeweils bis zu 8 Kohlenstoffatomen bedeuten, die gegebenenfalls durch Halogen, Hydroxy oder Aryl mit 6 bis 10 Kohlenstoffatomen substituiert sind, |
| T, V, X und Y | gleich oder verschieden sind und ein Sauerstoff- oder Schwefelatom bedeuten, |
| $R^5$ und $R^8$ | gleich oder verschieden sind und Wasserstoff, Halogen, Cycloalkyl mit 3 bis 8 Kohlenstoffatomen oder geradkettiges oder verzweigtes Alkyl oder Alkenyl mit jeweils bis zu 8 Kohlenstoffatomen bedeuten, die gegebenenfalls durch Cycloalkyl mit 3 bis 8 Kohlenstoffatomen, oder durch einen 5- bis 6-gliedrigen, aromatischen, gegebenenfalls benzokondensierten Heterocyclus mit bis zu 3 Heteroatomen aus der Reihe S, N und/oder O, oder durch Aryl mit 6 bis 10 Kohlenstoffatomen substituiert sind, wobei die Cyclen ihrerseits bis zu 3-fach gleich oder verschieden durch einen 5- bis 6-gliedrigen aromatischen Heterocylcus mit bis zu 3 Heteroatomen aus der Reihe S, N und/oder O, oder durch Phenyl, Benzyl, Halogen, Hydroxy, Carboxyl oder durch geradkettiges oder verzweigtes Alkyl, Alkoxy oder Alkoxycarbonyl mit jeweils bis zu 6 Kohlenstoffatomen substituiert sein können, oder Aryl mit 6 bis 10 Kohlenstoffatomen oder einen 5- bis 7-gliedrigen aromatischen, gegebenenfalls benzokondensierten Heterocyclus mit bis zu 3 Heteroatomen aus der Reihe S, N und/oder O bedeuten, die gegebenenfalls bis zu 3-fach gleich oder verschieden durch Halogen, Phenyl, Trifluormethyl, Hydroxy, Carboxyl oder durch geradkettiges oder verzweigtes Alkyl, Alkoxy oder Alkoxycarbonyl mit jeweils bis zu 6 Kohlenstoffatomen oder durch eine Gruppe der Formel $-(CO)_a-NR^9R^{10}$substituiert sind, worin |
| a | eine Zahl 0 oder 1 bedeutet, |
| $R^9$ und $R^{10}$ | gleich oder verschieden sind und Wasserstoff, Phenyl oder geradkettiges oder verzweigtes Alkyl oder Acyl mit jeweils bis zu 5 Kohlenstoffatomen bedeuten, |
| D und E | gleich oder verschieden sind und für Wasserstoff, Halogen, Trifluormethyl, Hydroxy, Carboxyl oder für geradkettiges oder verzweigtes Alkyl, Alkoxy oder Alkoxycarbonyl mit jeweils bis zu 6 Kohlenstoffatomen stehen, |
| $R^1$ | für Wasserstoff oder Cycloalkyl mit 3 bis 8 Kohlenstoffatomen steht, oder für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen, Phenyl oder durch einen 5- bis 6-gliedrigen aromatischen Heterocyclus mit bis zu 3 Heteroatomen aus der Reihe S, N und/oder O substituiert sind, oder für Phenyl oder einen 5- bis 6-gliedrigen aromatischen Heterocylcus mit bis zu 3 Heteroatomen aus der Reihe S, N und/oder O steht, wobei die Ringsysteme gegebenenfalls bis zu 3-fach gleich oder verschieden durch Halogen, Phenyl, Trifluormethyl oder geradkettiges oder verzweigtes Alkyl oder Alkoxy mit jeweils bis zu 5 Kohlenstoffatomen, Hydroxy oder durch eine Gruppe der Formel $-NR^{11}R^{12}$ substituiert sind, worin |
| $R^{11}$ und $R^{12}$ | die oben angegebene Bedeutung von $R^9$ und $R^{10}$ haben und mit dieser gleich oder verschieden sind, |
| L | für ein Sauerstoff- oder Schwefelatom steht, |

$R^2$ für Mercaptyl, Hydroxy, geradkettiges oder verzweigtes Alkoxy mit bis zu 8 Kohlenstoffatomen oder für die Gruppe der Formel

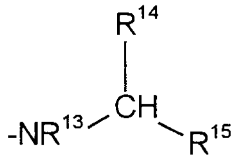

steht,
worin

$R^{13}$ Wasserstoff oder geradkettiges oder verzweigtes Alkyl mit bis zu 4 Kohlenstoffatomen bedeutet,

$R^{14}$ Wasserstoff, Phenyl oder einen 5- bis 6-gliedrigen aromatischen Heterocyclus mit bis zu 3 Heteroatomen aus der Reihe S, N und/oder O bedeutet,

$R^{15}$ Wasserstoff oder geradkettiges oder verzweigtes Alkyl mit bis zu 8 Kohlenstoffatomen bedeutet, das gegebenenfalls durch Hydroxy substituiert ist,

und deren Salze.

**2.** Substituierte Xanthine der Formel nach Anspruch 1, in welcher

A für einen Rest der Formel

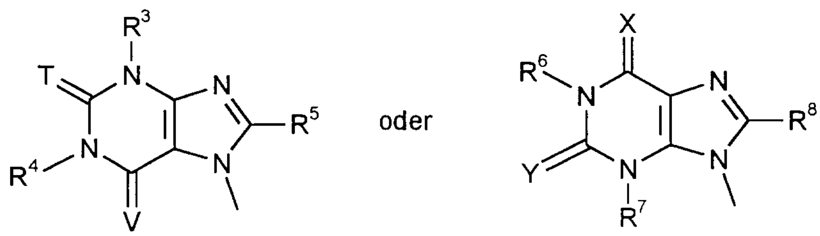
oder

steht,
worin

$R^3$, $R^4$, $R^6$ und $R^7$ gleich oder verschieden sind und
Wasserstoff, Phenyl, Cyclopropyl, Cyclopentyl oder Cyclohexyl bedeuten, oder geradkettiges oder verzweigtes Alkyl oder Alkenyl mit jeweils bis zu 6 Kohlenstoffatomen bedeuten, die gegebenenfalls durch Fluor, Chlor, Brom, Hydroxy oder Phenyl substituiert sind,

T, V, X und Y gleich oder verschieden sind und
ein Sauerstoff- oder Schwefelatom bedeuten,

$R^5$ und $R^8$ gleich oder verschieden sind und
Wasserstoff, Fluor, Chlor, Brom, Cyclopropyl, Cyclobutyl, Cyclopentyl, Cyclohexyl, Cycloheptyl oder für geradkettiges oder verzweigtes Alkyl oder Alkenyl mit jeweils bis zu 6 Kohlenstoffatomen bedeuten, die gegebenenfalls durch Cyclopropyl, Cyclopentyl, Cyclohexyl, Cycloheptyl, Naphthyl, Phenyl, Pyridyl, Thienyl oder Furyl substituiert sind, die ihrerseits bis zu 2-fach gleich oder verschieden durch Phenyl, Benzyl, Fluor, Chlor, Brom, Hydroxy oder durch geradkettiges oder verzweigtes Alkyl oder Alkoxy mit jeweils bis zu 4 Kohlenstoffatomen substituiert sein können, oder
Phenyl, Pyridyl, Thienyl oder Furyl bedeuten, die gegebenenfalls bis zu 2-fach

gleich oder verschieden durch Fluor, Chlor, Brom, Phenyl, Trifluormethyl, Hydroxy oder durch geradkettiges oder verzweigtes Alkyl oder Alkoxy mit jeweils bis zu 4 Kohlenstoffatomen oder durch eine Gruppe der Formel $-(CO)_a-NR^9R^{10}$ substituiert sind,
worin

| | |
|---|---|
| a | eine Zahl 0 oder 1 bedeutet, |
| $R^9$ und $R^{10}$ | gleich oder verschieden sind und<br>Wasserstoff, Phenyl oder geradkettiges oder verzweigtes Alkyl oder Acyl mit jeweils bis zu 4 Kohlenstoffatomen bedeuten, |
| D und E | gleich oder verschieden sind und<br>für Wasserstoff, Fluor, Chlor, Brom, Trifluormethyl, Hydroxy oder geradkettiges oder verzweigtes Alkyl oder Alkoxy mit jeweils bis zu 4 Kohlenstofatomen stehen, |
| $R^1$ | für Wasserstoff, Cyclopropyl, Cyclobutyl, Cyclopentyl, Cyclohexyl, Cycloheptyl oder für geradkettiges oder verzweigtes Alkyl oder Alkenyl mit jeweils bis zu 6 Kohlenstoffatomen steht, die gegebenenfalls durch Cyclopropyl, Cyclopentyl, Cyclohexyl, Phenyl, Pyridyl oder Thienyl substituiert sind, oder<br>für Phenyl, Pyridyl, Furyl oder Thienyl steht, die gegebenenfalls bis zu 2-fach gleich oder verschieden durch Fluor, Chlor, Brom, Phenyl, Trifluormethyl oder geradkettiges oder verzweigtes Alkyl oder Alkoxy mit jeweils bis zu 4 Kohlenstoffatomen oder durch eine Gruppe der Formel $-NR^{11}R^{12}$ substituiert sind,<br>worin |
| $R^{11}$ und $R^{12}$ | die oben angegebene Bedeutung von $R^9$ und $R^{10}$ haben und<br>mit dieser gleich oder verschieden sind, |
| L | für ein Sauerstoff- oder Schwefelatom steht, |
| $R^2$ | für Mercaptyl, Hydroxy, geradkettiges oder verzweigtes Alkoxy mit bis zu 6 Kohlenstoffatomen oder für die Gruppe der Formel |

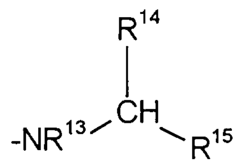

steht,
worin

| | |
|---|---|
| $R^{13}$ | Wasserstoff oder geradkettiges oder verzweigtes Alkyl mit bis zu 4 Kohlenstoffatomen bedeutet, |
| $R^{14}$ | Wasserstoff, Phenyl, Pyridyl, Furyl oder Thienyl bedeutet, |
| $R^{15}$ | Wasserstoff oder geradkettiges oder verzweigtes Alkyl mit bis zu 6 Kohlenstoffatomen bedeutet, das gegebenenfalls durch Hydroxy substituiert ist, |

und deren Salze.

**3.** Substituierte Xanthine der Formel nach Anspruch 1, in welcher

| | |
|---|---|
| A | für einen Rest der Formel |

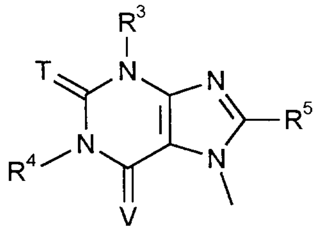

oder

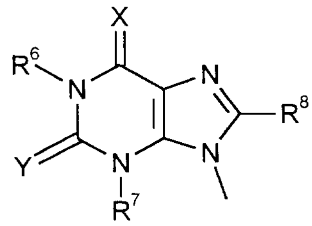

steht,
worin

$R^3$, $R^4$, $R^6$ und $R^7$ gleich oder verschieden sind und Wasserstoff, Phenyl, Cyclopropyl, Cyclopentyl oder Cyclohexyl bedeuten, oder geradkettiges oder verzweigtes Alkyl oder Alkenyl mit jeweils bis zu 5 Kohlenstoffatomen bedeuten, die gegebenenfalls durch Fluor, Chlor, Brom, Hydroxy oder Phenyl substituiert sind,

T, V, X und Y gleich oder verschieden sind und ein Sauerstoff- oder Schwefelatom bedeuten,

$R^5$ und $R^8$ gleich oder verschieden sind und Wasserstoff, Fluor, Chlor, Brom, Cyclopropyl, Cyclobutyl, Cyclopentyl, Cyclohexyl, Cycloheptyl oder für geradkettiges oder verzweigtes Alkyl oder Alkenyl mit jeweils bis zu 5 Kohlenstoffatomen bedeuten, die gegebenenfalls durch Cyclopropyl, Cyclopentyl, Cyclohexyl, Cycloheptyl, Phenyl, Pyridyl, Thienyl oder Furyl substituiert sind, die ihrerseits bis zu 2-fach gleich oder verschieden durch Phenyl, Benzyl, Fluor, Chlor, Brom, Hydroxy oder durch geradkettiges oder verzweigtes Alkyl oder Alkoxy mit jeweils bis zu 3 Kohlenstoffatomen substituiert sein können, oder Phenyl, Pyridyl, Thienyl oder Furyl bedeuten, die gegebenenfalls bis zu 2-fach gleich oder verschieden durch Fluor, Chlor, Brom, Phenyl, Trifluormethyl, Hydroxy oder durch geradkettiges oder verzweigtes Alkyl oder Alkoxy mit jeweils bis zu 3 Kohlenstoffatomen oder durch eine Gruppe der Formel $-(CO)_a-NR^9R^{10}$ substituiert sind,
worin

a eine Zahl 0 oder 1 bedeutet,

$R^9$ und $R^{10}$ gleich oder verschieden sind und Wasserstoff, Phenyl oder geradkettiges oder verzweigtes Alkyl oder Acyl mit jeweils bis zu 3 Kohlenstoffatomen bedeuten,

D und E gleich oder verschieden sind und für Wasserstoff, Fluor, Chlor, Brom oder Trifluormethyl stehen,

$R^1$ für Wasserstoff, Cyclopropyl, Cyclobutyl, Cyclopentyl, Cyclohexyl, Cycloheptyl oder für geradkettiges oder verzweigtes Alkyl oder Alkenyl mit jeweils bis zu 5 Kohlenstoffatomen steht, die gegebenenfalls durch Cyclopropyl, Cyclopentyl, Cyclohexyl, Phenyl, Pyridyl oder Thienyl substituiert sind, oder
für Phenyl, Pyridyl, Furyl oder Thienyl steht, die gegebenenfalls bis zu 2-fach gleich oder verschieden durch Fluor, Chlor, Brom, Phenyl, Trifluormethyl oder geradkettiges oder verzweigtes Alkyl oder Alkoxy mit jeweils bis zu 3 Kohlenstoffatomen oder durch eine Gruppe der Formel $-NR^{11}R^{12}$ substituiert sind,
worin
$R^{11}$ und $R^{12}$ die oben angegebene Bedeutung von $R^9$ und $R^{10}$ haben und mit dieser gleich oder verschieden sind,

L für ein Sauerstoff- oder Schwefelatom steht,

$R^2$ für Mercaptyl, Hydroxy, geradkettiges oder verzweigtes Alkoxy mit bis zu 5 Kohlenstoffatomen oder für die Gruppe der Formel

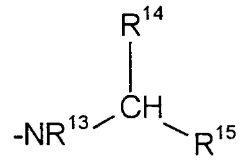

steht,
worin

$R^{13}$ Wasserstoff oder geradkettiges oder verzweigtes Alkyl mit bis zu 3 Kohlenstoffatomen bedeutet,

$R^{14}$ Wasserstoff, Phenyl, Pyridyl oder Thienyl bedeutet,

$R^{15}$ Wasserstoff oder geradkettiges oder verzweigtes Alkyl mit bis zu 3 Kohlenstoffatomen bedeutet, das gegebenenfalls durch Hydroxy substituiert ist,

und deren Salze.

**4.** Substituierte Xanthine der Formel nach Anspruch 1, in welcher

A für einen Rest der Formel

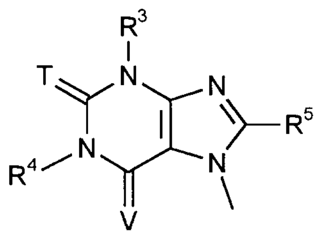

oder

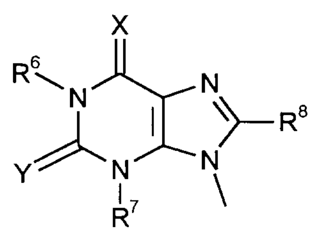

steht,
worin

$R^3$, $R^4$, $R^6$ und $R^7$ Wasserstoff, geradkettiges oder verzweigtes Alkyl mit jeweils bis zu 4 Kohlenstoffatomen bedeuten,

T, V, X und Y gleich oder verschieden sind und
ein Sauerstoff- oder Schwefelatom bedeuten,

$R^5$ und $R^8$ gleich oder verschieden sind und
Wasserstoff, Fluor, Chlor, Brom, Cyclopropyl, Cyclopentyl, Cyclohexyl oder für geradkettiges oder verzweigtes Alkyl oder Alkenyl mit jeweils bis zu 3 Kohlenstoffatomen bedeuten, die gegebenenfalls durch Cyclopropyl, Cyclopentyl, Cyclohexyl, Phenyl, Pyridyl, Thienyl oder Furyl substituiert sind, die ihrerseits bis zu 2-fach gleich oder verschieden durch Phenyl, Benzyl, Fluor, Chlor, Brom, Hydroxy oder durch geradkettiges oder verzweigtes Alkyl oder Alkoxy mit jeweils bis zu 3 Kohlenstoffatomen substituiert sein können, oder
Phenyl, Pyridyl, Thienyl oder Furyl bedeuten, die gegebenenfalls bis zu 2-fach gleich oder verschieden durch Fluor, Chlor, Brom, Phenyl, Trifluormethyl, Hydroxy oder durch geradkettiges oder verzweigtes Alkyl oder Alkoxy mit jeweils bis zu 3 Kohlenstoffatomen oder durch eine Gruppe der Formel $-(CO)_a-NR^9R^{10}$ substituiert sind,

worin

a — die Zahl 0 bedeutet,

$R^9$ und $R^{10}$ — gleich oder verschieden sind und Wasserstoff, oder geradkettiges oder verzweigtes Alkyl mit jeweils bis zu 3 Kohlenstoffatomen bedeuten,

D und E — gleich oder verschieden sind und für Wasserstoff, Fluor, Chlor, Brom oder Trifluormethyl stehen,

$R^1$ — für Wasserstoff, Cyclopropyl, Cyclopentyl, Cyclohexyl, Cycloheptyl, Phenyl oder für geradkettiges oder verzweigtes Alkyl mit bis zu 5 Kohlenstoffatomen steht,

L — für ein Sauerstoffatom steht,

$R^2$ — für Hydroxy, geradkettiges oder verzweigtes Alkoxy mit bis zu 5 Kohlenstoffatomen oder für die Gruppe der Formel

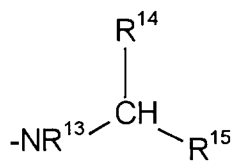

steht,
worin

$R^{13}$ — Wasserstoff oder geradkettiges oder verzweigtes Alkyl mit bis zu 3 Kohlenstoffatomen bedeutet,

$R^{14}$ — Phenyl bedeutet,

$R^{15}$ — geradkettiges oder verzweigtes Alkyl mit bis zu 3 Kohlenstoffatomen bedeutet, das gegebenenfalls durch Hydroxy substituiert ist,

und deren Salze.

**5.** Substituierte Xanthine nach Anspruch 1 bis 4 zur therapeutischen Anwendung.

**6.** Verfahren zur Herstellung von substituierten Xanthinen nach Anspruch 1 bis 4, dadurch gekennzeichnet, daß man zunächst durch Umsetzung von
Verbindungen der allgemeinen Formel (II)

$$A\text{-}H \qquad (II)$$

in welcher

A die oben angegebene Bedeutung hat,

mit Verbindungen der allgemeinen Formel (III)

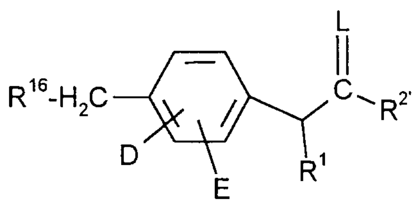

(III)

in welcher

D, E, L und $R^1$ die oben angegebene Bedeutung haben,

$R^{16}$ für Hydroxy oder Halogen, vorzugsweise für Brom steht,

und

$R^{2'}$ für geradkettiges oder verzweigtes Alkoxy mit bis zu 8 Kohlenstoffatomen steht,

in inerten Lösemitteln und in Anwesenheit von Basen und/oder Hilfsmitteln in Verbindungen der allgemeinen Formel (Ia)

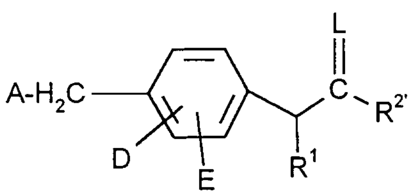

(Ia)

in welcher

A, D, E, L, $R^1$ und $R^{2'}$ die oben angegebene Bedeutung haben,

überführt und diese gegebenenfalls ($R^2$ = OH) verseift,
oder gegebenenfalls diese Säuren mit Glycinolen und Glycinolderivaten der allgemeinen Formel (IV)

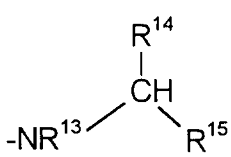

(IV)

in welcher

$R^{13}$, $R^{14}$ und $R^{15}$ die oben angegebene Bedeutung haben,

in inerten Lösemitteln, gegebenenfalls in Anwesenheit von Basen und/oder Hilfsmitteln umsetzt.

**7.** Arzneimittel, enthaltend mindestens ein substituiertes Xanthin nach Anspruch 1 bis 4.

**8.** Arzneimittel nach Anspruch 7 zur Behandlung von Atherosklerose.

**9.** Verfahren zur Herstellung von Arzneimitteln nach Anspruch 7 und 8, dadurch gekennzeichnet, daß man die substituierten Xanthine gegebenenfalls mit geeigneten Hilfs- und Trägerstoffen in eine geeignete Applikationsform überführt.

**10.** Verwendung der substituierten Xanthine nach Anspruch 1 bis 4 zur Herstellung von Arzneimitteln.

Europäisches Patentamt

# EUROPÄISCHER RECHERCHENBERICHT

Nummer der Anmeldung
EP 96 11 4577

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | GB-A-2 276 383 (MERCK & CO INC)<br>* Seite 148 - Seite 197; Ansprüche *<br>--- | 1-10 | C07D473/00<br>C07D473/06<br>C07D473/08<br>A61K31/52 |
| A | EP-A-0 363 320 (CIBA-GEIGY AG)<br>* Seite 22 - Seite 33; Ansprüche *<br>----- | 1-10 | |

RECHERCHIERTE SACHGEBIETE (Int.Cl.6)
C07D

Der vorliegende Recherchenbericht wurde für alle Patentansprüche erstellt

| Recherchenort | Abschlußdatum der Recherche | Prüfer |
|---|---|---|
| DEN HAAG | 2.Januar 1997 | Luyten, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)